(19) Europäisches Patentamt
European Patent Office
Office européen des brevets

(11) Publication number: 0 013 008
B1

(12) EUROPEAN PATENT SPECIFICATION

(45) Date of publication of patent specification: 16.03.83

(21) Application number: 79105298.8

(22) Date of filing: 20.12.79

(51) Int. Cl.³: C 07 C 29/15, C 07 C 31/20, C 07 C 31/04, C 07 C 31/08, C 07 C 69/003, C 07 C 67/36

# (54) Process for producing alcohols.

(30) Priority: 21.12.78 US 971667
21.12.78 US 971816
15.11.79 US 91242

(43) Date of publication of application: 09.07.80 Bulletin 80/14

(45) Publication of the grant of the patent: 16.03.83 Bulletin 83/11

(84) Designated Contracting States: BE DE FR GB IT NL SE

(56) References cited:
DE - A - 2 644 185
DE - B - 1 092 458
FR - A - 2 234 256
GB - A - 655 237
US - A - 2 535 060
US - A - 2 549 470
US - A - 2 636 049
US - A - 3 989 799
US - A - 4 170 605

(73) Proprietor: UNION CARBIDE CORPORATION
Old Ridgebury Road
Danbury Connecticut 06817 (US)

(72) Inventor: Dombek, Bernard Duane
1631 Woodvale Drive
Charleston, West Virginia (US)

(74) Representative: Schmied-Kowarzik, Volker, Dr. et al,
Patentanwälte Dr. V. Schmied-Kowarzik Dipl.-Ing. G. Dannenberg Dr. P. Weinhold Dr. D. Gudel Dipl.-Ing. S. Schubert Dr. P. Barz Siegfriedstrasse 8
D-8000 München 40 (DE)

EP 0 013 008 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

(56) References cited

ERDOL UND KOHLE — ERDGAS — PETROCHEMIE VEREINIGT MIT BRENNSTOFFCHEMIE, vol. 32, no. 7, July 1979 pages 313—316 Industrieverlag von Hernhaussen KG
Leinfelden, DE.
A. DELUZARCHE et al.: "Ractions between Co and H2"

HIGH PRESSURE SCIENCE AND TECHNOLOGY, 6th AIRCRAFT Conference 1979, pages 733—738, Plemun Press
New York, U.S.A.
R. FONSECA et al.: "High Pressure Synthesis of polyalcohols by catalytic hydrogenation of carbon monoxide"

CATALYTICA LETTERS, vol. 5, no. 1, January 1979, published by Catalytica Associates, pages 1—3
Santa Clara, California, U.S.A.
"Coal conversion"

JOURNAL OF AMERICAN CHEMICAL SOCIETY, vol. 101, no. 24, 21st November 1979, pages 7419—7421 J. S. BRADLEY: "Homogeneous Carbon Monoxide hydrogenation to Methanol Catalyzed by Soluble Ruthenium Complexes"

CONFERENCE OF PROFESSOR W. KEIM, University of Achen cited page 16, lines 22—23 in the present patent application

# Process for producing alcohols

This invention relates to an improved process, and the catalyst which achieves this process, for making ethylene glycol, methanol and ethanol directly from synthesis gas i.e., mixtures of hydrogen and carbon monoxide. More particularly, this invention achieves the production of ethylene glycol directly from synthesis gas using a ruthenium carbonyl complex catalyst under process conditions which heretofore were regarded as being incapable of producing ethylene glycol with a ruthenium containing catalyst. This invention encompasses a process of producing ethylene glycol, methanol and ethanol directly from the reaction of synthesis gas in the presence of a stable ruthenium catalyst. The process of this invention is distinctive in the stability of the process, avoiding any significant loss of ruthenium values from reaction. In addition, this process features a unique ruthenium containing catalyst, possibly mononuclear, for the catalytic process which produces ethylene glycol, methanol and ethanol.

In view of the prior national applications BE—PS 877 448, FR—PS 2 430 404, GB—PS 2 024 811, NL—OS 79 05 114, DE—OS 2 941 998 the applicant has voluntarily limited the scope of the present application and submitted separate claims for Belgium, France, United Kingdom, the Netherlands and the Federal Republic of Germany.

## DISCUSSION OF THE PRIOR ART

Pruett and Walker, U.S. Patent No. 3,833,634, patented September 3, 1974, based on an application originally December 21, 1971, describe a process for preparing glycols by reacting an oxide of carbon with hydrogen using a rhodium carbonyl complex catalyst. The examples of the patent compare the reaction of hydrogen and carbon monoxide in the presence of the desired rhodium containing catalyst and other metals. In Example 9 of the patent, the reaction was attempted with triruthenium dodecacarbonyl as the catalyst using tetrahydrofuran as the solvent with a reaction temperature of 230°C, for 2 hours, and "the product contained no polyhydric alcohol." As will be shown below, Pruett and Walker apparently failed because they did not run at the conditions of reaction long enough and/or with enough ruthenium containing catalyst to achieve reaction to produce at least a detectable amount of a polyhydric alcohol such as ethylene glycol; see Example 82, *infra*. Example 82 employs substantially more ruthenium than did Pruett and Walker in their Example 9. Unquestionably, ruthenium is not as active a catalyst source to produce glycol as is rhodium under the conditions investigated.

Gresham, U.S. Patent No. 2,535,060, describes a process for preparing monohydric alcohols by introducing carbon monoxide, hydrogen and a hydroxylated solvent into a reaction vessel and heating the mixture in the presence of a ruthenium-containing substance and an alkaline reagent which controls the pH within the range of 7 to 11.5, at a temperature within the range of 150° to 300°C under a pressure within the range of 200 to 1,000 bar.

Solid ruthenium dioxide is used in Examples 1 and 2 of the Gresham patent. Experimental evidence (see Example 83, *infra*) shows that Gresham utilized a heterogeneous reaction system.* At column 2, lines 30—33 of the patent, the patentee states his belief that ruthenium dioxide is reduced *in situ* during the reaction. Example 1 compares the use of a number of solutes such as phosphoric acid, acidic phosphate buffer, no solutes at all, ammonia and sodium bicarbonate. In this example, the solvent was water. In Example 2 of Gresham, a number of alcohols were characterized as solvents.

Gresham states that ruthenium and its compounds are "specific" in their effect upon this reaction and other catalysts "do not lead to straight chain primary alcohols under the conditions of this process". There is no indication that Gresham's process, as operated by him, produced ethylene glycol.

Gresham's work should be contrasted with his earlier work described in U.S. Patent No. 2,636,046, filed October 16, 1948. In this patent, Gresham describes the production of polyfunctional oxygen-containing organic products including such compounds as ethylene glycol, glycerine, and the like**. This is accomplished by the reaction of hydrogen with carbon monoxide in the presence of a solvent to produce glycol. According to this patent, the reaction of carbon monoxide with hydrogen must be at pressures of above 1,000 bar and "particularly above a minimum of about 1,400 bar in order to obtain the "polyfunctional oxygen-containing organic compounds . . . in excellent yield" (column 2, lines 9—17).

The patent specifically states at column 2, lines 37—43, that

"(I)n the hydrogenation of oxides of carbon at pressures of 1,000 bar and below, virtually no polyfunctional compounds are produced. At pressures above 1,000 bar and especially at pressures of about 1,500 to 5,000 bar preferably 2,000 to 5,000 bar, polyfunctional compounds are obtained."

---

*See discussion of Howk, et al, *infra*, and refer to *Catalysis*, Vol. 2, 1978, pp. 67—68, published by The Chemical Society, London WIV 0BN, England.

**Note the evaluation of this work by Rathke and Feder, JACS, *100*, pp. 3623—3625 (May 24, 1978).

Though the examples of the patent describe the use only of cobalt catalyst, the patentee, at column 3, line 61, indicates that the catalyst may contain "cobalt, ruthenium, etc". According to the patentee, the most outstanding results are obtained by using a catalyst containing cobalt, especially compounds of cobalt which are soluble in at least one of the ingredients of the reaction mixture.

According to Roy L. Pruett, *Annals, New York Academy of Sciences*, Vol. 295, pages 239—248 (1977), at page 245, metals other than rhodium were tested to determine the production of ethylene glycol from mixtures of carbon monoxide and hydrogen. These metals include cobalt, ruthenium, copper, manganese, iridium and platinum. Of these metals, only cobalt was found to have a slight activity, citing British Patent 665,698 which corresponds generally to the last mentioned Gresham U.S. Patent. Pruett stated that such slight activity with cobalt was "qualitatively" in agreement with the results obtained by Ziesecke, 1952, Brennstoff-Chem, *33*:385.

Prior to the filling of U.S. Patent No. 2,535,060 and subsequent to the filling of U.S. Patent No. 2,636,046, there was filed on April 12, 1949, a commonly assigned application by Howk, et al which issued as U.S. Patent No. 2,549,470 on April 17, 1951. The Howk, et at patent is directed to a catalytic process for making monohydric straight chain alcohols and does not mention the production of ethylene glycol. The patent emphasizes the production of straight chain primary hydroxyalkanes having from 3 to 50 or more carbon atoms in the molecule. This, the patent states, is accomplished by introducing hydrogen, carbon monoxide and a hydroxylated solvent into a reaction vessel, and heating the mixture in the presence of a catalyst of the class consisting of ruthenium metal, ruthenium oxide and ruthenium carbonyl, at a pressure within the range of 200 to 1,000 bar and at a temperature within the range of 100° to 250°C. The liquid hydroxyl-containing reaction medium may be water or alcohol, preferably a primary hydroxyalkane having from 1—10 carbon atoms per molecule. According to the patentee, a substantial proportion of the reaction product usually consists of alcohols containing more than 6 carbon atoms per molecule. The patent goes on to state (column 1, line 50, et seq.):

"The reaction products usually contain virtually no hydrocarbons, acids, esters, or branched-chain alcohols. These results were entirely unexpected, in view of the existing knowledge of the catalytic reaction between carbon monoxide and hydrogen in the presence of alcohols and Group VIII metal catalysts."

According to the Howk, et al patent:

"It should be emphasized here that under the conditions of temperature, pressure and gas ratios just described, no reaction takes place between carbon monoxide and hydrogen in a liquid medium (water or alcohol) if one of the common group VIII metals, such as cobalt or nickel, is used as the catalyst. This is evidenced by the fact that, using, for example, a cobalt catalyst, no significant drop in pressure is observed when carbon monoxide and hydrogen are contacted under the conditions recited. Ruthenium is thus unexpectedly different from these related metals." (Column 4, lines 19—30).

The numbered examples indicate an apparent preference for making normal-monohyric alcohols, with the proportion of pentane soluble to pentane insoluble alcohol being at least 2:1. In one example, starting at the bottom of column 6 of Howk, et al, the solvent employed is characterized as a carboxylic acid or anhydride rather than the neutral hydroxylated solvents which were described in the other examples. This comparative example demonstrated that in a process operated at 200°C for 18 hours using pressures maintained in the range of 300—950 bar by repressurizing periodically with synthesis gas, there was produced a reaction product containing "a large quantity of wax". According to the author, 40.55 parts of esters boiling from 59°C at atmospheric pressure to 150°C at 166 154 mbar pressure were obtained and this can be compared to the wax obtained in the amount of 37.06 parts. In that particular example, the patentee appears to have demonstrated that when one does not employ the hydroxylated solvent, the amount of wax essentially equals the amount of pentane soluble alcohol products obtained. This is supported by the statement at column 2 of Gresham U.S. 2,535,060 which refers to Howk, et al.

At column 3, lines 54 et seq., Howk, et al describe the influence that pressure has on the course of the reaction. According to Howk, et al with pressures up to about 150 bar the reaction products are only hydrocarbons. This appears to be in accord with recent work described by Masters, et al in German Patent Application (Offenlegungsschrift) 2,644,185*, based upon British priority application Specification No. 40,322—75, filed October 2, 1975. Masters, et al obtained only hydrocarbons at such pressures using a ruthenium catalyst.

Fenton, U.S. Patent No. 3,579,566, patented May 18, 1971, is concerned with a process of reducing organic acid anhydrides with hydrogen in the presence of a Group VIII noble metal catalyst and a biphyllic ligand of phosphorus, arsenic or antimony. The process of Fenton bears a remarkable similarity to oxo processing conditions to produce aldehydes and alcohols (compare with Oliver, et al, U.S. Patent No. 3,539,634, patented November 10, 1970) except that Fenton fails to supply an olefinic

*See Doyle, et al, *J. of Organometallic Chem.*, *174*, C55—C58 (1979), who conclude that the process characterized in the German OLS involved a heterogeneous Fischer-Tropsch reaction.

compound to the reaction. In the Fenton reaction, and acid anhydride, such as acetic acid anhydride is reduced to ethylidene diacetate in the presence of hydrogen and a rhodium halide or a mixture of palladium chloride and ruthenium trichloride catalyst, provided in combination with triphenylphosphine. Ethylene glycol diacetate is also observed. Carbon monoxide, which is added to some of the examples of Fenton, is described by Fenton, at column 2, lines 48—51, as follows: "If desired, a suitable *inert* gas, such as carbon monoxide can also be charged to the reaction zone . . .". (Emphasis added). Of particular significance is the fact that none of Fenton's examples produce a methyl ester, as are produced by the process of copending U.S. Patent Application S.N. 971,667, discussed below and encompassed herein. Another point is that Fenton's ethylidene diacetate can be thermally cracked to produce vinyl acetate, see column 1, lines 42—44. It would seem possible that such occurred in Example 1 of Fenton and it is further possible that acetic acid added to the vinyl acetate to form ethylene glycol diacetate.

The following is believed to be a fair analysis of the aforementioned references, i.e., what they teach one skilled in the art and the direction that they could lead one in pursuit of whatever is their objectives:

(1) Gresham, U.S. Patent No. 2,636,046 states that at exceedingly high pressures in excess of 1,500 bar, one can produce some glycol and glycol esters by the reaction of carbon monoxide and hydrogen utilizing, most desirably, a cobalt catalyst although some undescribed ruthenium compound can be substituted for cobalt.

(2) The Pruett and Walker patent makes a showing in Examples 9 and 17 at columns 11 and 12, respectively, that the reaction of CO and $H_2$ in the presence of ruthenium carbonyl and cobalt carbonyl complexes operated at about 1309—1722 bar (19,000—25,000 psi) pressure will, in the case of ruthenium, produce no polyhydric alcohols and, in the case of cobalt, produce trace amounts of mono and diacetates of ethylene glycol. Thus, with respect to the cobalt catalyst a minimum pressure of about 1309 bar (19,000 psi) seems to be needed to make any glycol compound. In the case of ruthenium, the pressure at which glycol can be made from CO and $H_2$ had not been defined.

(3) Howk, et al (U.S. Patent No. 2,549,470) who employ a lower pressure reaction than Gresham (U.S. 2,636,046) produce only monohydric alcohols from the reaction of CO and $H_2$ using a solid ruthenium catalyst.* The maximum pressure for the Howk, et al process is about 1,000 bar. The reaction produces a spectrum of monohydric alcohols ranging from methanol to very high molecular weight alcohols, some alcohols containing up to 40 carbon atoms. The products are classified as pentane soluble materials and pentane insoluble materials. The pentane insoluble higher alcohols are characterized as waxes and less desirable than the pentane soluble alcohols. When Howk, et al ran the reaction in acetic acid at a pressure ranging from 300 to 950 bar, there was produced "a large quantity of wax together with a liquid". The amount of wax was essentially the same amount, in parts by weight, as ester products, assumed to be esters of monohydric alcohols.

(4) The second Gresham Patent (U.S. Patent No. 2,535,060) appears to be an improvement on the Howk, et al patent. It describes the desirability of controlling the pH of the reaction medium in the reaction between carbon monoxide and hydrogen in the presence of a ruthenium-containing catalyst such as described by Howk, et al. The presence of trace amounts of carboxylic acid is considered very undesirable by Gresham. Gresham states that traces of carboxylic acids produce an acidity which "has a very profound effect upon the subsequent course of the reaction, causing the formation of relatively longer chain products, such as waxy alcohols containing up to 50 or more carbon atoms per molecule. If the pH is more strongly acidic, high molecular weight waxy products are formed in still greater proportions." The copending application referred to is the Howk, et al., U.S. Patent No. 2,549,470, mentioned previously. Thus, Gresham specifies that it is desirable to maintain the pH of the reaction solution alkaline in order to obtain a better distribution of straight chain monohydric primary alcohols. According to Gresham, the quantity of methanol formed in his reaction "is extremely small" (see column 1, line 49).

(5) There is apparently a minimum pressure according to Howk, et al used to avoid the formation of hydrocarbons and this appears to be supported by the disclosure of Masters, et al, *supra*. However, in view of Doyle, et al, *supra*, there may be a greater similarity in the processes of Howk, et al and Masters, et al.

(6) The choice of metal catalyst and the appropriate conditions for such kinds of reactions are not predictable. For example Pruett, et al, the Gresham patents, Howk, et al, and Pruett, state that many metals do not function as catalysts in the reactions they are concerned with.

(7) Fenton utilized rhodium, palladium and ruthenium halides in the presence of a mixture of hydrogen and carbon monoxide and an acid anhydride, and recognized only the reduction of the anhydride.

*See *Catalysis, supra,* who class the Howk, et al catalytic process as heterogeneous, and Example 83, *infra,* which supports such classification.

In a recent report (Catalytica Letters, Vol. 5, No. 1, January 1979, published by Catalytica Associates, Inc., 3255 Scott Blvd., Suite 7—E, Santa Clara, California 95051), J. S. Bradley (in Paper No. 54 of the 1st Int. Symp. on Homogeneous Catalysis, held at Corpus Christi, Texas on November 29 through December 1, 1978) of Exxon Corporation, produced methanol and methyl formate at a selectivity greater than 99% without hydrocarbon products detected, by the reaction of synthesis gas ($H_2$:CO=2:1) under pressures "on the order of 1,300 bar" and at "temperatures around 270°C", using "a Ru catalyst, which under the conditions of the reaction was present as $Ru(CO)_5$." Bradley reported according to this report that "no homologation products were found". According to a separate unpublished report, Bradley stated that he reacted 10 millimoles of ruthenium tris(acetylacetonate) in tetrahydrofuran with synthesis gas ($H_2$:CO=2:1) pressurized to 1,400 bar (20,580 psi) at 268°C and obtained about 0.75 moles of methanol, about 0.15 moles of methyl formate, and no observed amounts of ethanol, ethylene glycol, acetates or hydrocarbons. Compare with result with that found by Pruett and Walker, *supra*, and the work of Fonseca, et al and Williamson, et al, *infra*.

An interesting exception to the previously reported inactivity of ruthenium catalysts to produce glycol is the high pressure (viz. 1650—1750 bars) experiment reported by R. Fonseca, et al, High Pressure Science and Technology, 6th AIRAPT Conference (Chapt. "High Pressure Synthesis of Polyalcohols by Catalytic Hydrogenation of Carbon Monoxide"), pages 733—738 (1979), published by Plenum Press, New York. In this experiment the authors report the reaction in tetraglyme of a CO:$H_2$ (1:2 ratio) mixture at 1650—1750 bar, i.e., about 1722 bar (25,000 psi) and at 230°C using triruthenium dodecacarbonyl and 2-pyridinol as a ligand, both in unstated amounts, for a period of 5 hours. The authors report a % conversion of 12.9 (unstated basis), a % yield of polyols of 3 (unstated basis), and % selectivities as follows: ethylene glycol, 22.9; glycerine, 0; and methanol, 16.1. Further, in Williamson, et al, United States Patent 4,170,605, patented October 9, 1979, in patentees report in Examples I and II the reaction in 1-propanol of synthesis gas (CO:$H_2$ = 1:1) at 1723 bar (25,000 psig) and at 230°C using ruthenium tris(acetylacetonate) and 2-hydroxypyridine, the latter being the same ligand employed by Fonseca et al, *supra*, for a period of 2 and 3 hours, respectively. In Example I, Williamson, et al report the production of 4 grams of product* containing (mole percent basis): ethylene glycol, 57; and methanol, 25. In Example II, 7 grams of product* are reported containing 66 and 16 mole percent of ethylene glycol and methanol, respectively. The similarity between the high pressure experiment reported by Fonseca, et al and the high pressure examples of the Williamson, et al patent is further apparent from the standpoint that neither Fonseca, et al nor Williamson, et al report formation of glycerine** or ethanol.

As pointed out above, ethylene glycol can be produced directly from a mixture of hydrogen and carbon monoxide using a rhodium carbonyl complex as a catalyst. The literature describes (see U.S. Patent No. 3,957,857, issued May 18, 1976) that a desirable rhodium compound can be in the form of a rhodium carbonyl cluster compound, particularly one which exhibits a particular 3-band infrared spectral pattern. There has been a substantial amount of work done on the formation of ethylene glycol

---

*Included in the 4 and 7 grams of product are trace amounts of water and methylformate, as well as 16 mole % (Example I) and 15 mole % (Example II) of propylformate. The latter compound would appear to be derived from 1-propanol initially present in the reaction mixture, rather than a synthesis gas derived product.

**See Deluzarche, et al, *Erdöl and Kohle-Ergas-Petrochemi vereinigt mit Brennstoff-Chemie*, Bd. 32, Heft 7, July 1979; pp. 313—316, where it is shown that pressures over 1722 bar (3625 bars) are needed to produce glycerol, whereas the instant process produces glycerol at below 1033 bar (15,000 psi). Professor W. Keim, Univ. Aachen, Aachen, W. Germany, has orally reported the reaction of CO and $H_2$ in the presence of $Ru_3(CO)_{16}$ (this formula seems to be in error and should be $Ru_3(CO)_{12}$) to produce glycol but had not reported all of the reaction conditions, especially the pressures that he employed. It is understood that Prof. Keim has reported that pressures greater than 1,000 bar are needed to produce glycol directly from synthesis gas with any metal carbonyl complex. Note the similarity between Keim's position and that recited in Gresham, U.S. Patent No. 2,636,046, *supra*.

from mixtures of hydrogen and carbon monoxide in the presence of rhodium carbonyl clusters (see the list of patents and applications recited in footnoted Table A below*).

The above discussion provides a detailed characterization of technology heretofore published or filed upon which relates to the direct production of ethylene glycol from mixtures of carbon monoxide and hydrogen or the production of monohydric alcohols from the direct reaction of hydrogen and carbon monoxide in the presence of a ruthenium catalyst. For the purposes of the discussion and descriptions contained herein, mixtures of hydrogen and carbon monoxide, regardless of the amount of each present, will be characterized, for the sake of convenience, as "synthesis gas". Thus, mole ratios of hydrogen to carbon monoxide of e.g. 40 to 1 and .05 to 1 are arbitrarily classified as "synthesis gas". Where the molar ratio of one to the other is significant to the invention herein described, then specific reference to the desired molar ratio will be made.

THE PROBLEM

Owing to the limited availability of petroleum sources the cost of producing chemicals from petroleum has been steadily increasing. Many have raised the dire prediction of significant oil shortages in the future. Obviously a different low cost source is needed which can be converted into the valuable chemicals now derived from petroleum sources. Synthesis gas is one such source which can be effectively utilized in certain circumstances to make chemicals.

The most desirable aspect of synthesis gas is that it can be produced from non-petroleum sources. Synthesis gas is derived by the combustion of any carbonaceous material including coal, or any organic material, such as hydrocarbons, carbohydrates and the like. Synthesis gas has for a long time been considered a desirable starting material for the manufacture of a variety of chemicals. A number of chemicals have been made commercially from synthesis gas. Hydrocarbons have been made by the Fischer-Tropsch catalytic reaction. Methanol is commercially manufactured by a heterogeneous catalytic reaction from synthesis gas. Aldehydes and alcohols are made from the reaction of olefins and synthesis gas. If one could expand the production of chemicals in a commercial manner from synthesis gas then one would not be as presently dependent upon petroleum as the basic raw material even though it is an excellent raw material for making synthesis gas.

There is described herein a process which has wide ranging possibilities for the production of a host of valuable chemicals. The process of this invention involves the conversion of synthesis gas, however derived, into a limited variety of valuable alcohol compounds which themselves can be directly consumed or which can be employed as starting materials to make other valuable chemicals. The process of this invention is concerned with making 2 carbon atom alcohols, to wit, ethanol and ethylene glycol and in particular, ethylene glycol. In addition, the process of this invention also produces

---

* TABLE A:

| | |
|---|---|
| U.S.P. 3,833,634 | Patented September 3, 1974 |
| U.S.P. 3,878,214 | Patented April 15, 1975 |
| U.S.P. 3,878,290 | Patented April 15, 1975 |
| U.S.P. 3,878,292 | Patented April 15, 1975 |
| U.S.P. 3,886,364 | Patented May 27, 1975 |
| U.S.P. 3,929,969 | Patented December 30, 1975 |
| U.S.P. 3,940,432 | Patented February 24, 1976 |
| U.S.P. 3,944,588 | Patented March 16, 1976 |
| U.S.P. 3,948,965 | Patented April 16, 1976 |
| U.S.P. 3,952,039 | Patented April 20, 1976 |
| U.S.P. 3,957,857 | Patented May 18, 1976 |
| U.S.P. 3,968,136 | Patented July 6, 1976 |
| U.S.P. 3,974,259 | Patented August 10,1976 |
| U.S.P. 3,989,799 | Patented November 2, 1976 |
| U.S.P. 4,001,289 | Patented January 4, 1977 |
| U.S.P. 4,013,700 | Patented March 22, 1977 |
| U.S.P. 4,111,975 | Patented September 5, 1978 |
| U.S.P. 4,115,428 | Patented September 19, 1978 |
| U.S.P. 4,115,433 | Patented September 19, 1978 |
| U.S.P. 4,133,776 | Patented January 9, 1979 |
| U.S.P. 4,151,192 | Patented April 24, 1979 |
| U.S.P. 4,153,623 | Patented May 8, 1979 |
| U.S.P. 4,162,261 | Patented July 24, 1979 |

large amounts of methanol. The process of this invention is capable of producing predominantly ethylene glycol or predominantly methanol, or predominantly ethanol, or mixtures of them each in large concentrations. The process of this invention provides the capability of a low cost route to methanol, ethanol and ethylene glycol, especially ethylene glycol.

One of the deficiencies of the aforementioned processes for making ethylene glycol from synthesis gas utilizing a rhodium carbonyl complex catalyst is the enormous price of rhodium. Rhodium presently is employed in catalytic converters which comprise the automotive combustion devices for reducing automotive pollutant emissions. The high cost of rhodium is created by its limited availability and the tremendous demand for it. Thus, a commercial process which uses rhodium as a catalyst is affected by the high capital expense to purchase the metal and the strict controls needed to limit catalyst losses in order to keep the economics of the process competitive.* Ruthenium, on the other hand, is a precious metal which has no significant commercial application. Its present cost is approximately 1/20th and less than that of rhodium even though its concentration in the ore from which both are obtained is about the same. Ruthenium has been explored as a catalyst by many. It has been considered as a hydrogenation catalyst, as a hydroformylation catalyst, as a catalyst to produce a wide range of monohydric alcohols (non-specific as to any of them) exclusive of methanol, as an alcohol homologation catalyst such as for the conversion of methanol to ethanol,** as a high pressure catalyst to selectively produce methanol and methyl formate, and its inactivity has been noted as a catalyst to produce glycol, see above.

THE INVENTION

This invention relates to processes and catalysts for selectively making the products methanol ethylene glycol and ethanol, or derivative precursors such as acylates, directly from the reaction of hydrogen and carbon monoxide. The process comprises:

(a) establishing and maintaining a solvent-containing liquid phase comprising solubilized ruthenium carbonyl complex in which the solvent has a dielectric constant of at least 2, determined at 25°C or at its melting point, whichever is higher;

(b) supplying hydrogen and carbon monoxide in said liquid phase; and

(c) maintaining said liquid phase for a sufficient period of time at a temperature and pressure which causes said hydrogen and carbon monoxide to react to produce such products, said temperature is between about 50°C and 400°C and said pressure is between 34,5 bar (500 psia) and 1033 bar (15,000 psia). The catalyst of this invention is that catalyst which is formed during the course of the reaction.

Further details of the invention are recited below.

COMPARISON OF INVENTION WITH CITED ART

The process of this invention is distinguishable from the aforementioned reported work and disclosures of others in the following ways:

(1) As to Gresham, U.S. 2,535,060, *supra*, the present invention employs a homogeneous liquid phase catalytic reaction in which the catalyst is dissolved in a liquid phase medium, not a heterogeneous reaction as employed by Gresham. In addition, Gresham and Howk et al, *supra*, produce only straight chain alcohols, offering little selectivity to any of them, and the quantity of methanol formed in Gresham's reaction "is extremely small".

(2) As to Gresham, U.S. 2,636,046, *supra*, a minimum pressure of 1400 bar is required to form polyfunctional oxygen-containing organic compounds as compared with a maximum pressure of about 1033 bar (15,000 psi) for this invention. Gresham fails to show that ruthenium in any particular form functions as an effective catalyst at even his high pressures. It is believed that Gresham's high pressure requirements make his process commercially uneconomical, particularly when considered in the light of the amount of glycol produced with his preferred cobalt catalyst (see Pruett and Walker, *supra*, Example 17).

(3) Pruett, *supra*, and Pruett et al, *spura*, establish the view that ruthenium carbonyl complexes would not function to produce ethylene glycol, even at extremely high pressures, viz. 1,300—1,700 bar. This is supported by Bradley, *supra*, who effected the reaction at 1,300 bar and obtained no ethylene glycol.

(4) R. Fonseca, et al, *supra*, and Williamson, et al, *supra*, which are the only art citations which produced any ethylene glycol using a ruthenium-containing catalyst, performed their experiments at the extreme pressure of about (25,000 psi). Neither Fonseca, et al nor Williamson, et al, however, report

---

*See Cornils, et al, Hydrocarbon Processing, June, 1975, pp. 83 to 91.

**See, for example, U.S. Patents 4,133,966 and 3,285,948; and Japanese Patent Application (Kokai) No. 52—73804/77 (June 21, 1977) (Application No. 50—149391/75 (application date, December 15, 1975)) to Mitsubishi Gas Chemical Industry Company.

the formation of ethanol or glycerine. The instant process, on the other hand, is effected at pressures below 1033 bar (15,000 psi) and, in addition to providing ethylene glycol at such substantially lower pressures, provides ethanol as well as glycerine.

FURTHER DISCUSSION OF THE INVENTION

This process constitutes a relatively low pressure process for selectively converting synthesis gas to such valuable chemicals as ethylene glycol, ethanol and methanol. Also produced by the process of this invention are glycerol (i.e. glyercine), 1,2-propylene glycol, 1-propanol and methyl formate. However, the process of this invention is mainly concerned with the production of ethylene glycol (the most valued product), ethanol and methanol since they are produced in significantly greater amounts than the other products. This process is capable of being oriented to enhance the selectivity in favor of any one of the methanol, ethanol and ethylene glycol. An added feature of this invention is the ability to enhance the productivity, when desired, of such by-products as glycerol. The process of this invention is accomplished even when the predominant products of the reaction are derivatives such as methyl carboxylates, ethyl carboxylates and ethylene glycol mono- and dicarboxylates.

The process of this invention is carried out with the ruthenium carbonyl complex dissolved in a solvent, even though such complex may exist during the reaction in more than one liquid phase. In this sense, the reaction is termed a homogeneous liquid phase reaction. There may be more than one such phase existing in the reaction zone but the ruthenium carbonyl complex existing as the catalyst is always dissolved in at least one of such phases and is always in a dissolved liquid state. The problem with heterogeneous ruthenium catalysis in the reaction zone is that such will induce the Fischer-Tropsch reaction resulting in the formation of hydrocarbons and/or a variety of oxygenated hydrocarbons having a variety of molecular weights with low selectivity to any one compound. In fact the presence of such products suggests that undissolved ruthenium is present.

The process of this invention involves the solubilization of ruthenium in the presence of synthesis gas at temperatures, pressures and for a period of time sufficient to produce ethylene glycol. Such conditions are set forth herein. In simplistic and in the broadest terms, the invention comprises the solubilization under the reaction conditions (i.e., time, temperature and pressure) of a ruthenium source, preferably ruthenium in the absence of any other platinum group metal (viz., platinum, palladium rhodium, and iridium),* in an appropriate solvent, preferably one which has a dielectric constant of at least 2 determined at 25°C or at its melting point, whichever is the higher value, under a prescribed synthesis gas pressure. The reaction conditions comprise (i) a period of time at a temperature and pressure which cause the hydrogen and carbon monoxide to react to produce the desired products, (ii) a temperature between 50°C and 400°C and (iii) a pressure between 34,5 bar (500 psia) and 1033 bar (15,000 psia). The catalyst of this invention is the ruthenium containing carbonyl complex which under the prescribed reaction conditions catalyzes the aforementioned reaction between carbon monoxide and hydrogen.

The process of this invention is distinctive in the selection of materials which comprise the homogeneous liquid phase mixture, the reaction parameters and the stability of the ruthenium containing catalyst in most cases, indeed, in all cases studied. As with any technology, this process has undergone evolutionary changes and its further examination will undoubtedly bring about more changes, most likely in the form of additional or substitutional steps and/or materials.

In the preferred form of the invention the process is carried out in the presence of a promoter. A promoter, in the context of this invention, is a material provided to the reaction which provides a promotional effect in that it enhances the production (viz., rate, yield or efficiency) of any of the products, or it improves the selectivity of the reaction toward ethylene glycol rather than methanol or ethanol, or it improves the selectivity of the reaction to ethanol rather than methanol irrespective of the amount of ethylene glycol produced, or it helps to reduce the loss of ruthenium during the reaction. A promoter may be any Lewis base containing compound. Any Lewis base may be a promoter but all Lewis bases will not serve to act as a promoter under any given set of reaction conditions. The effectiveness of the Lewis base as a promoter will in large measure be dependent upon the reaction conditions selected. Operation of the process in the absence of the Lewis base promoter will result in most instances in less productivity and therefore, exploitation of the process in a commercial sense will probably necessitate the use of a promoter.

The amount of Lewis base promoter added to the process is that amount which provides the promotional effect. The maximum amount employed is that amount whose presence is too costly for the economical operation of the process, or substantially reduces the promotional effect without any advantage, or provides no advantage in the operation of the process, or a combination of these factors. The promoter can be a material used in miniscule quantities to a material employed in maximum

---

*See U.S. Patent 3,989,799, patented November 2, 1976, wherein ruthenium is a cation in a mixed metal rhodium-containing carbonyl complex.

quantities such as a solvent for the reaction and the ruthenium carbonyl complex catalyst. Indeed, the promoter can be a material such as carboxylic acids, which when present react with the products of the reaction.

Apart from the conditions of the reaction in terms of time, temperature and pressure, the selection of solvent and optionally the Lewis base promoter constitute important considerations in the most advantageous practice of this invention. The selections of solvent and the promoter are not narrowly limited yet there appears to be some degree of cooperation that each imparts to the success of the process and the selection of one oftentimes dictates the selection of the other in order to maximize the benefits of the invention.

It is found necessary that there be used a solvent that is capable of maintaining the chosen ruthenium carbonyl complex and, optionally the Lewis base promoter (if it is not the solvent), in the homogeneous liquid phase mixture throughout the reaction. This appears to be the prime function of the solvent. The solvent may possibly provide an additional benefit such as influencing the kinds of ion pairing that exist during the course of the reaction.

The catalyst of this invention is a ruthenium compound which contains carbon monoxide directly bonded to ruthenium (ruthenium carbonyl). The ruthenium compound which is provided to the reaction is not necessarily in a form which will effectively catalyze the reaction even if it contains a carbon monoxide ligand bonded to it. Ruthenium compounds such as ruthenium salts, oxides and carbonyl clusters may be introduced to the reaction in a condition which allows them to be solubilized, and under the conditions of the reaction they are converted into a carbonyl complex which effectively catalyzes the reaction. That is why they are defined in terms of products made by the process. The composition and structure of the ruthenium carbonyl complex which catalyzes the desired reaction is not specifically known. It may be a monoruthenium or polyruthenium compound. Illustrative of polyruthenium compounds are the well-known cluster compounds of ruthenium. However, the addition of a cluster containing only a carbonyl ligand such as $Ru_3(CO)_{12}$ does not alone create the catalyst and as such cause the catalytic reaction. Some modification of such structure is needed, possibly the destruction of the cluster structure to a mononuclear ruthenium structure. Factors in achieving the catalyst are the reaction parameters, the choice of solvent and, optionally, the Lewis base promoter that one employs. Because varied reaction conditions and solvents, with and without promoters, result in different amounts of the desired products of the process, and different rates, efficiences and/or yields, it is presumed that each provides a different and distinct catalytic environment.

The ruthenium-containing substances which may be employed in the practice of this invention to form the catalyst under process conditions encompass those which are described, for example, in Gresham, U.S. Patent No. 2,535,060, at column 2, starting at line 38 to line 48, and ruthenium carbonyl compounds. It is not advisable to place ruthenium compounds or substances on a support material for use in the process of this invention because such offers no benefits over solubilizing such ruthenium compounds in combination with the aforementioned solvent and Lewis base promoter. Moreover, ruthenium deposited on a support material can be expected to be solubilized in the homogeneous liquid phase reaction system of this invention as it is contacted with carbon monoxide. Even ruthenium metal in the presence of the solvent, carbon monoxide and hydrogen can be converted to a ruthenium carbonyl complex which is soluble. Ruthenium oxides, such as dioxide, sesquioxide, or tetraoxide, are capable under appropriate conditions of being solubilized and converted to a carbonyl complex which can be used to form the catalyst under the conditions of this process. However, when using such insoluble ruthenium compounds, they must first be solubilized before the effective operation of the process of this invention. Ruthenium carbonyl compounds (which include ruthenium carbonyl hydrides or ruthenium carbonyl clusters) are already provided with a carbonyl ligand, and under the conditions of the reaction can be sufficiently changed to achieve the desired catalytic effect. Ruthenium salts such as those of organic acids can be employed in the practice of this invention to produce the catalyst. In addition to those ruthenium compounds described in the aforementioned Gresham patent, one may employ ruthenium compounds of bidentate ligands, allyl complexes, arene complexes, halides, and alkyl complexes. The choice of ruthenium compounds is varied and not critical to this invention. A number of ruthenium complexes are known to be more stable to the presence of carbon monoxide than other ruthenium compounds and the skilled worker can determine which particular ruthenium compound might take longer to initiate a reaction than other ruthenium compounds. On that basis, one can select for the purposes of convenience the particular ruthenium compound to be utilized in forming the catalyst. However, ruthenium which is associated with an organic molecule or complexed with carbon monoxide is most readily solubilized so as to provide the ruthenium catalyst of this process.

As characterized above, this process is operated as a homogeneous liquid phase mixture. The process is typically carried out in a solvent for the catalyst and the Lewis base promoter, when added. Thus the solvent is a liquid in which the catalyst (presumed to be a ruthenium carbonyl complex) and the added Lewis base promoter are soluble under the prescribed conditions of the reaction. The solvent may be solid at room temperature but should at least in part be a liquid under the conditions of reaction.

A preferred solvent is a liquid at reaction conditions which is polar or complexes ions. Of the polar solvents those which have a relatively high dielectric constant are more preferred. As for the solvents

which complex ions, the desirable solvents are those which under the reaction conditions have the capacity of complexing ions such as available cations. As stated previously, the solvent may provide the Lewis base component. Solvents having a dielectric constant at 25°C or at its melting temperature, whichever is higher, of greater than 2 are preferred.

Illustrative of suitable polar solvents are, e.g., water, ketones, esters including lactones, amides including lactams, sulfones, sulfoxides, halogenated hydrocarbons or aromatic hydrocarbons. Illustrative of specific solvents encompassed by the above classes of polar solvents are, for example, aromatic hydrocarbons, e.g., benzene, toluene, xylene, naphthalene, alkylnaphthalene; carboxylic acids such as acetic acid, propionic acid, butyric acid, caproic acid, stearic acid, benzoic acid, cyclohexanecarboxylic acid; ketones such as acetone, methyl ethyl ketone, cyclohexanone, cyclopentanone; esters such as methyl acetate, ethyl acetate, propyl acetate, butyl acetate, methyl propionate, ethyl butyrate, methyl laurate; anhydrides such as phthalic anhydride, acetic anhydride; lactams such as N-alkyl caprolactam, such as N-methylcaprolactam, N-alkyl pyrrolidinones such as N-methyl pyrrolidinone; cyclic ureas such as N,N'-dimethylimidazolidone; polyols such as ethylene glycol, glycerine, erythritol, polyalkylene glycol containing two to about ten thousand repeating units; lactones such as gamma-butyrolactone; halogenated hydrocarbons such as chlorobenzene, chloroform, methylene chloride, 2,2-dichloropropane; amides such as diemthylformamide, dimethylacetamide, hexamethylphosphoramide; sulfones such as sulfolane, dimethylsulfone, substituted sulfolanes, sulfoxides such as dimethylsulfoxide, diphenyl sulfoxide; as well as many others.

Illustrative of suitable complexing solvents are e.g. the ethers or, cryptands. Illustrative of specific solvents encompassed by the above classes of complexing solvents are, for example, ethers such as tetrahydrofuran, tetrahydropyran, diethyl ether, 1,2-dimethoxybenzene, 1,2-diethoxybenzene, the mono and dialkyl ethers of alkylene and polyalkylene glycols, such as ethylene glycol, of 1,2-propylene glycol, of 1,2-butylene glycol, of diethylene glycol, of di-1,2-propylene glycol, of triethylene glycol, of pentaethylene glycol (such as triglyme, tetraglyme and pentaglyme), of di-1,2-butylene glycol or of oxyethyleneoxypropylene glycols, preferably those in which the alkylene group contains 2 and/or 3 carbon atoms in the divalent moiety, such as ethylene and 1,2-propylene; the cryptands such as described in U.S. Patent No. 4,111,975, which description of cryptands, as promoters in that case, are incorporated herein by reference; the crown ethers (or Crown Ethers, as one may prefer) such as described in U.S. Patent No. 4,162,261, which description of crown ethers, as solvents in that case, are incorporated herein by reference; as well as many others.

The choice of solvent in any particular case can be a complex decision. Some solvents such as the carboxylic acids play a dual role in the practice of the process of this invention. They can provide the required Lewis base promoter as well as the solvent. Other solvents which can play this dual function include, e.g., the crown ethers and the cryptands, as well as many others. In many instances, solvents react with the products of the reaction and such reactive solvents are considered useful in the practice of this invention because the derivative products obtained are an excellent source for the desired products of the reaction. For example, the carboxylic acids are not only effective solvents and promoters, they are also reactive with ethylene glycol, methanol and ethanol products, to produce ethylene glycol dicarboxylates, methyl carboxylates, and ethyl carboxylates. These carboxylates can be readily hydrolyzed to produce the alcohol products. This is not necessarily an uneconomical method to produce such products (for example, Halcon International Inc., New York, N.Y., is constructing a plant to produce ethylene glycol by the reaction of acetic acid and ethylene and the hydrolysis of the resulting ethylene diacetate). In many cases (and possibly in the preferred cases) another Lewis base promoter will be employed in combination with a solvent which has the capacity to serve in such dual function. This is because such other Lewis base promoter is found to be more effective in generating the desired products when used in combination with that solvent under the conditions of reaction chosen.

An important class of solvents contemplated in the practice of this invention are mixtures of the aforementioned polar solvents and the complexing solvents. Various polar solvents mixed with other polar or complexing solvents are contemplated to provide enhanced results either in terms of rates, selectivity, conversions and/or yields of one or more of the desired products. Which mixtures will achieve what results has not been determined. Combinations of, e.g., sulfolane with crown ethers, lactones, amides or ureas are contemplated as potentially useful. Combinations of, e.g., crown ethers with lactones, amides, and ureas are contemplated as potentially useful.

The Lewis bases suitable as promoters in the practice of this process are not a narrowly defined class of materials. They encompass a broad range of inorganic and organic materials, and all members of the class are contemplated as employable in the practice of this invention. Its effectiveness in some instances can be noted when used in as little an amount which is the least amount that a measurable promotional effect is seen to an amount wherein the Lewis base is also a solvent for the reaction. The Lewis base can serve a dual function by playing the role as the solvent for the reaction. There is no simple way of determining what Lewis base will function effectively under a given set of reaction conditions. In the typical case, when a Lewis base exhibits promotional affects on the rate of the reaction, it is present and dissolved in the liquid phase in a range of from .01 mole to $10^6$ moles for each atom (gram atomic weight) of ruthenium present in the reaction. More preferred, the Lewis base is present (even when the solvent used is a Lewis base) in the liquid phase in a range from 1 mole to $10^4$

moles for each atom of ruthenium present in the reaction; most preferably, greater than one mole up to 1000 moles of the Lewis base for each atom of ruthenium present and dissolved in the liquid phase.

The Lewis base promoters include inorganic as well as organic compounds. Illustrative of suitable organic compounds are those containing at least one Lewis base nitrogen atom or at least one Lewis base oxygen atom or a combination of such nitrogen and oxygen atoms. The carbon atoms can be part of an acylic and/or cyclic radical such as aliphatic, cycloaliphatic and aromatic carbon radicals. Usually, the organic Lewis bases contain at least 2 carbon atoms and no more than 40 carbon atoms. The Lewis base nitrogen atoms are usually in the form of imino (—N=), amino (—N—) and nitrilo (N≡), etc. The Lewis base oxygen atoms can be in the form of groups such as hydroxyl (aliphatic or phenolic), carboxyl

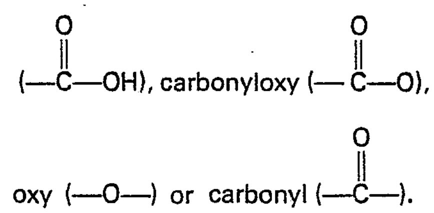


The organic Lewis bases may also contain other atoms and/or groups as substituents of the aforementioned radicals such as alkyl, aryl and chloro substituents. The Lewis base promoter also includes a variety of inorganic compounds such as, for example, inorganic amines and a variety of inorganic metal compounds.

Illustrative of suitable classes of Lewis base promoters are, for example, any of the following: monoamines and polyamines including those compounds in which Lewis base nitrogen forms part of a ring structure; alkanolamines; acyl compounds including aliphatic, cycloaliphatic and aromatic carboxylic acids, ester derivatives and anhydrides of such acids, usually having no more than 20 carbon atoms; bis(triorgano phosphine)iminium compounds; ketones; ethers; amides; crown ethers; cryptands, hydroxides and salts of various metals including, for example, carboxylates, halides, carbonates, bicarbonates, sulfates and bisulfates of any of the alkali metals, alkaline earth metals as well as of other metals such as iron; as well as many other compounds which can function as Lewis bases or serve as a source for the Lewis base under reaction conditions.

Illustrative of specific Lewis bases are the following: methyl-, ethyl-, isopropyl- and octylamines; dimethyl-, diisoamyl- and diisobutylamines; methylethylamine; trimethyl- and triethylamines; methyldiethylamine; triisobutyl- and tridecylamines; 1,2-ethanediamine; 1,3-propanediamine; diethylenetramine; triethylenetetraamine; tetraethylenepentamine,

$$NH_2CH_2CH_2NHCH_2CH_2NHCH_2CH_2NHCH_2CH_2NH_2;$$

N,N,N',N'-tetramethylethylenediamine, $(CH_3)_2NCH_2CH_2N(CH_3)_2$; N-pentamethyldiethylenetriamine; *p*-phenylenediamine; *o*-tolidene; aniline; 1-naphththyl- and 2-naphthylamines; *p*-toluidine; benzylamine; diphenylamine; dimethylaniline; bis-(1,8)-dimethylaminonaphthalene; cyclohexylamine; dicyclohexylamine; piperidine and N-methylpiperidine; 3-phenylpiperidine; pyridine and 2-methylpyridine; 2,4,6-trimethylpyridine; 2-dodecylpyridine; 2-aminopyridine; 2-(dimethylamino)pyridine; quinoline; 2-(dimethylamino)-6-methoxyquinoline; pyrimidine; 1,8-phenanthroline; piperazine; N-methyl- and N-ethylpiperazines; 2,2'-bipyridyl and alkyl-substituted 2,2'-bi-pyridyls; 1,4-diazabicyclo (2.2.2)octane ("triethylenediamine"); hexamethylenetetraamine; purine; isopropanolamine; diethanolamine; di-n-propanolamine; triethanolamine; triisopropanolamine; bis(dimethylaminoethyl)ether; N,N-dimethylglycine; N-methyliminodiacetic acid; 2-hydroxypyridine; 2-methoxypyridine; 2,6-dimethoxypyridine; 4-methyl-2-hydroxypyridine; 4-methyl-2,6-dihydroxypyridine; morpholine; N-methyl- and N-ethylmorpholines; hexadecylmorpholine; ethylenedimorpholine; tetraethylenedimorpholine; picolinic acid; nitrilotriacetic acid; 2,5-dicarboxypiperazine; N-(2-hydroxyethyl)-iminodiacetic acid; 2,6-dicarboxypyridine; ammonia; hydroxylamine; hydrazine; hexamethylphosphoramide; dimethylformamide; n-methylpyrrolidone; acetic acid; propionic acid; butyric acid; 2,2,6,6-tetramethylheptane-3,5-dione, $(CH_3)_3(CC(O)CH_2C(O)C(CH_3)_3$; sulfolane; 18-crown-6; 15-crown-5; tetrahydrofuran; diphenylether; bis(triphenylphosphine)iminium chloride, $[(C_6H_5)_3P]_2N^+Cl^-$; bis(triphenylphosphine)iminium iodide, $[(C_6H_5)_3P]_2N^+I^-$; cesium formate; sodium acetate; sodium sulfate; potassium carbonate; potassium bicarbonate; cesium oxide; cesium hydroxide, potassium hydroxide, magnesium bromide; calcium iodide; cesium bromide; sodium fluoride; potassium fluoride; rubidium bromide; cesium iodide; rubidium iodide; potassium iodide; sodium iodide; sodium bromide; lithium iodide; lithium bromide; lithium chloride; potassium chloride; lithium diethylamine; sodium phenyl; butyllithium; cobalt diiodide, e.g. $CoI_2{\cdot}2H_2O$; tetracarbonyl cobaltate anion $[Co(CO)_4]^{-1}$; ferrous iodide, e.g. $FeI_2{\cdot}4H_2O$.

Not all of the above Lewis bases, or for that matter all Lewis bases, will necessarily function effectively in all of the embodiments of the process of this invention. In most cases a degree of selection between the choice of Lewis base, the amount of ruthenium, the choice of solvent and the

reaction parameters will be required to obtain the level of productivity sought.

Because $H_2$ is supplied to the reaction, a hydride of ruthenium can exist in the reaction system. There is no appreciation of the particular role that hydride is playing in the reaction. It is believed that either too much or too little hydrogen present in the reaction will not favor the production of ethylene glycol. In such a case, one can contemplate a role for hydride in the reaction mechanisms occurring.

Though the process of this invention is capable of providing a combination of ethylene glycol, ethanol and methanol, in many instances one or more of them is formed as a minor component only. Because ethylene glycol is the most valued of the products, its production obviously makes this process attractive. By the same reasoning, ethanol's higher market value than methanol also enhances the commercial attractiveness of this process. A process which produces the same amount of ethylene glycol and produces more ethanol will have commercial attractiveness, assuming all other factors are equal.

At this time, no particular basis has been found for predicting whether any particular set of process conditions and reactants encompassed by this invention will produce ethanol except those that have already been established by experimentation. It has been found that certain process conditions do produce ethanol while others are not apparently as effective in producing ethanol. The ability to make ethanol may reside in the particular ruthenium catalyst, the Lewis base promoter (if employed), the solvent, and/or the temperature and pressure of reaction, but in all probability ethanol production is dependent on a combination of all of these.

The relative amounts of carbon monoxide and hydrogen which are initially present in the reaction mixture can be varied over a wide range. In general, the molar ratio of $CO:H_2$ is in the range of from 40:1 to 1:40, suitably from 20:1 to 1:20, and preferably from 10:1 to 1:10. It is to be understood, however, that molar ratios outside the broadest of these ranges may be employed. Substances or reaction mixtures which give rise to the formation of carbon monoxide and hydrogen under the reaction conditions may be employed instead of mixtures comprising carbon monoxide and hydrogen which are used in preferred embodiments in the practice of the invention. For instance, the product alcohols are contemplated as obtainable by using mixtures containing carbon dioxide and hydrogen. Mixtures of carbon dioxide, carbon monoxide and hydrogen can also be employed. If desired, the reaction mixture can comprise steam and carbon monoxide.

The quantity of catalyst employed is not narrowly critical and can vary over a wide range. In general, the process is deisrably conducted in the presence of a catalytically effective quantity of the active ruthenium species which gives a suitable and reasonable reaction rate. Reaction can proceed when employing as little as $1 \times 10^{-6}$ weight percent, and even lesser amounts, of ruthenium based on the total weight of reaction mixture (i.e., the liquid phase mixture). The upper concentration limit can be quite high, e.g., 30 weight percent ruthenium, and higher, and the realistic upper limit in practicing the invention appears to be dictated and controlled more by economics in view of the cost of ruthenium. Since the rate of conversion of synthesis gas may be dependent upon the concentration of ruthenium employed, higher concentrations achieving higher rates, then large concentrations may prove to be a most desirable embodiment of this invention. Depending on various factors such as the Lewis base promoter (if employed), the partial pressures of carbon monoxide and hydrogen, the total operative pressure of the system, the operative temperature, the choice of solvent, and other considerations, a catalyst concentration of from $1 \times 10^{-3}$ to 20 weight percent ruthenium (contained in the complex catalyst) based on the total weight of reaction mixture, is generally desirable in the practice of the invention.

The temperature which may be employed in practicing the process may vary over a wide range of elevated temperatures. In general, the process can be conducted at a temperature between 50°C and 400°C and higher. Temperatures outside this stated range, though not excluded from the scope of the invention, do not fall within certain desirable embodiments of the invention. At the lower end of the temperature range, and lower, the rate of reaction to desired product becomes markedly slow. At the upper temperature range, and beyond, catalyst, solvent, or Lewis base promoter instability may occur. Notwithstanding these factors, reaction will continue and the alcohols and/or their derivatives will be produced. Additionally, one should take notice of the equilibrium reaction for forming ethylene glycol:

$$2\ CO + 3H_2 = HOCH_2CH_2OH$$

At relatively high temperatures the equilibrium increasingly favors the left hand side of the equation. To drive the reaction to the formation of increased quantities of ethylene glycol, higher partial pressures of carbon monoxide and hydrogen are required. Processes based on correspondingly higher operative pressure, however, do not represent preferred embodiments of the invention in view of the high investment costs associated with erecting chemical plants which utilize high pressure utilities and the necessity of fabricating equipment capable of withstanding such enormous pressures. Preferred temperatures are between 100°C and 350°C, and most desirably between 150°C and 300°C.

The process is suitably effected over a wide superatmospheric pressure range. At pressures in the direction of and below 34,5 bar (500 psia) the rate of desired product formation is quite slow, and consequently, relatively faster reaction rates and/or higher conversions to the desired products can be

obtained by employing higher pressures, e.g., pressures of at least 69 bar (1,000 psia). The upper pressure limitation is 1033 bar (15,000 psia). Effecting the proçess below 1033 bar (15,000 psia) especially below 689 bar (10,000 psia), results in significant cost advantages which are associated with lower pressure equipment requirements and operating costs. A suitable pressure range is from 34,5 bar (500 psia) to 861 bar (12,500 psia). The pressures referred to about represent the total pressure of hydrogen and carbon monoxide.

The process is effected for a period of time sufficient to produce the desired alcohol products and/or derivatives thereof. In general, the residence time to produce the desired products can vary from minutes to a number of hours, e.g., from a few minutes to 24 hours, and longer. It is readily appreciated that the residence period (time) will be influenced to a significant extent by the reaction temperature, the concentration and choice of Lewis base promoter and ruthenium source, the total gas pressure and the partial pressure exerted by its components, the concentration and choice of solvent, and other factors. The synthesis of the desired product(s) by the reaction of hydrogen with carbon monoxide is suitably conducted under operative conditions which give reasonable reaction rates and/or conversions.

The process can be executed in a batch, semi-continuous, or continuous fashion. The reaction can be conducted in a single reaction zone or a plurality of reaction zones, in series or in parallel, or it may be conducted intermittently or continuously in an elongated tubular zone or series of such zones. The material of construction should be such that it is inert during the reaction and the fabrication of the equipment should be able to withstand the reaction temperature and pressure. The reaction zone can be fitted with internal and/or external heat exchanger(s) to thus control undue temperature fluctuations, or to prevent any possible "run-away" reaction temperatures due to the exothermic nature of the reaction. In preferred embodiments of the invention, agitation means to vary the degree of mixing of the reaction mixture can be suitably employed. Mixing induced by vibration, shaker, stirrer, rotatory, oscillation, ultrasonic, etc., are all illustrative of the types of agitation means which are contemplated. Such means are available and well-known to the art. The catalyst precursor may be initially introduced into the reaction zone batchwise, or it may be continuously or intermittently introduced into such zone during the course of the synthesis reaction. Means to introduce and/or adjust the reactants, either intermittently or continuously, into the reaction zone during the course of the reaction can be conveniently utilized in the process especially to maintain the desired molar ratios of and the partial pressures exerted by the reactants.

As intimated previously, the operative conditions can be adjusted to optimize the conversion of the desired product and/or the economics of the process. In a continuous process, for instance, when it is preferred to operate at relatively low conversions, it is generally desirable to recirculate unreacted synthesis gas with/without make-up carbon monoxide and hydrogen to the reactor. Recovery of the desired product can be achieved by methods well-known in the art such as by distillation, fraction-action, extraction, and the like. A fraction comprising ruthenium complex, generally contained in byproducts and/or the solvent, can be recycled to the reaction zone, if desired. All or a portion of such fraction can be removed for recovery of the ruthenium values or regeneration thereof, if necessary. Fresh ruthénium precursor, Lewis base promoter and/or solvent, can be intermittently added to the recycle stream or directly to the reaction zone, if needed.

Many embodiments of the ruthenium carbonyl complex, Lewis base promoter and solvent combinations encompassed by this invention are sufficiently stable to allow repeated use of the ruthenium carbonyl complex. This is especially noted when the promoter is an alkali metal halide, particularly and preferably an alkali metal iodide. For example, the process of this invention can be continuously operated in a pressure reactor which is continuously fed synthesis gas. The velocity of the synthesis gas is sufficient to strip products of the reaction out of the reactor leaving behind in the reactor the ruthenium carbonyl complex, Lewis base and solvent combination. The products are separated from the unreacted synthesis gas and the synthesis gas is recycled to the reactor. The products, in this embodiment, are recovered free of ruthenium, Lewis base and solvent. In this embodiment, the catalyst need not be removed from the reactor to a recovery zone for separating product. Thus a catalyst treatment step is avoided. The examples below depict batch reactions; however, the above continuous gas recycle process can be operated in a similar manner. That is, the batch reactor simulates the continuous reactor except for the gas sparging and continuous gas recycle.

Although this invention has been described with respect to a number of details, it is not intended that this invention should be limited thereby. Moreover, the examples which follow are intended solely to illustrate a variety, including the most favorable, embodiments of this invention and are not intended in any way to limit the scope and the intent of this invention.

EXAMPLES

In examples 1—4, recorded in Table I below, the following procedure was employed:

A 500 ml stainless steel bomb reactor containing a removable glass liner was charged with a mixture of $Ru_3(CO)_{12}$, solvent and Lewis base as designated below. Carbon monoxide and hydrogen were then added in the designated ratios to the reactor to attain a pressure therein of 208 bar at 25°C. The reactor was rocked and the contents heated to the reaction temperature and maintained at this temperature for two hours while rocking the reactor. The pressure was maintained at the specified

reaction pressure during the indicated period of the reaction. The reactor was then cooled and vented. The contents of the reactor were removed and analyzed by gas chromatography. Table I directly follows.

TABLE I

| Example Nos. | Millimoles of Ruthenium | Lewis base | Millimoles of Lewis base | Solvent | Milliliters of Solvent | Reaction Temperature, °C. | Reaction Pressure, bar | $H_2$/CO Ratio | Reaction Period, hours | Grams of Ethylene Glycol Recovered | Grams of Methanol Recovered | Grams of Ethanol Recovered |
|---|---|---|---|---|---|---|---|---|---|---|---|---|
| 1 | 2.34 | LiI | 14.9 | Sulf[1] | 40 | 230 | 345 | 1:1 | 2 | .10 | 4.25 | .25 |
| 2 | 2.34 | KI | 15.0 | Sulf | 40 | 230 | ,, | 1:1 | 2 | .16 | 5.76 | .31 |
| 3 | 2.34 | NaI | 15.0 | Sulf | 40 | 230 | ,, | 1:1 | 2 | .17 | 6.49 | .36 |
| 4 | 2.34 | NaI | 15.0 | NMP[2] | 50 | 230 | ,, | 1:1 | 2 | .16 | 4.12 | .09 |

[1] "Sulf" is an abbreviation for sulfolone.

[2] "NMP" is an abbreviation for N-methylpyrrolidinone.

The following procedure was employed in the examples recorded in Table II below;

A 150 ml capacity stainless steel reactor capable of withstanding pressures up to 3,000 bar was charged with a mixture of solvent, ruthenium as triruthenium dodecacarbonyl and Lewis base promoter, as indicated below. The reactor was sealed and charged with a gaseous mixture, containing carbon monoxide and hydrogen in the ratios specified below, to a pressure of 173 bar (2,500 psig). Heat was applied to the reactor and its contents; when the temperature of the mixture inside the reactor reached the designated reaction temperature recited below, as measured by a suitably placed thermocouple, addition of carbon monoxide and hydrogen ($H_2$:CO=designated mole ratio) was made to bring the pressure to the specified reaction pressure recited below. The temperature (in °C) was maintained at the desired value for the reported time. During this period of time additional carbon monoxide and hydrogen was added whenever the pressure inside the reactor dropped by more than 35,5 bar (500 psig). With these added repressurizations the pressure inside the reactor was maintained at the reaction pressure ± 35,5 bar (± 500 psig) over the entire reaction period.

After the reaction period, the vessel and its contents were cooled to room temperature, the excess gas vented and the reaction product mixture was removed. Analysis of the reaction product mixture was made by gas chromatographic analysis. Table II directly follows.

TABLE II

| Example Nos. | Millimoles of Ruthenium | Lewis base | Millimoles of Lewis base | Solvent | Milliliters of Solvent | Reaction Temperature, °C. | Reaction Pressure, bar | $H_2$/CO Ratio | Reaction Period, hours | Grams of Ethylene Glycol Recovered | Grams of Methanol Recovered | Grams of Ethanol Recovered[11] |
|---|---|---|---|---|---|---|---|---|---|---|---|---|
| 5 | – | LiI | 22.4 | Sulf[1'] | 75 | 230 | 345 | 1:1 | 4 | – | – | – |
| 6 | 3.51 | LiI | 22.4 | Sulf[1] | 75 | 230 | ,, | 1:1 | 4 | 1.33 | 3.69 | – |
| 7 | 3.51 | LiI | 22.4 | Sulf[1] | 75 | 230 | 862 | 1:1 | 1.15 | 1.42 | 5.36 | 0.14 |
| 8 | 3.51 | LiI | 22.4 | Sulf[1] | 75 | 230 | ,, | 1:1 | 1.20 | 1.17 | 5.44 | 1.06 |
| 9 | – | NaI | 18 | 18–C–6[2] | 75 | 230 | ,, | 1:1 | 4 | – | – | – |
| 10 | 3 | NaI | 6 | 18–C–6[2] | 75 | 230 | ,, | 1:1 | 1.75 | 1.68 | 6.26 | 1.0 |
| 11 | 3 | NaI | 18 | 18–C–6[2] | 75 | 230 | ,, | 1:1 | .83 | 1.38 | 7.91 | 1.06 |
| 12 | 3 | NaI | 36 | 18–C–6[2] | 75 | 230 | ,, | 1:1 | .47 | 1.23 | 7.63 | 0.81 |
| 13 | 3 | NaI | 18 | 18–C–6[2] | 75 | 260 | ,, | 1:1 | .33 | 1.20 | 8.30 | 1.88 |
| 14 | 3 | KI | 3 | 18–C–6[2] | 75 | 230 | ,, | 1:1 | 2.30 | 1.51 | 5.75 | 0.70 |
| 15 | 3 | KI | 6 | 18–C–6[2] | 75 | 230 | ,, | 1:1 | 1.50 | 1.38 | 6.53 | 0.71 |
| 16 | 3 | KI | 12 | 18–C–6[2] | 75 | 230 | ,, | 1:1 | .83 | 1.22 | 6.73 | 0.63 |
| 17 | 9 | KI | 12 | TG[3] | 75 | 230 | ,, | 1:1 | 2.83 | .86 | 6.27 | 0.81 |
| 18 | 3 | NaI | 6 | TG[3] | 75 | 230 | ,, | 1:1 | 4 | .48 | 6.28 | 1.08 |
| 19 | 3 | LiI | 24 | TG[3] | 75 | 260 | ,, | 1:1 | 1.30 | .31 | 3.42 | 3.40 |

TABLE II (Continued)

| Example Nos. | Millimoles of Ruthenium | Lewis base | Millimoles of Lewis base | Solvent | Milliliters of Solvent | Reaction Temperature, °C. | Reaction Pressure, bar | $H_2$/CO Ratio | Reaction Period, hours | Grams of Ethylene Glycol Recovered | Grams of Methanol Recovered | Grams of Ethanol Recovered[11] |
|---|---|---|---|---|---|---|---|---|---|---|---|---|
| 20 | 9 | KOAc[9] | 48 | TG[3] | 75 | 260 | 862 | 1:1 | 2.10 | .08 | 7.13 | — |
| 21 | 3 | $K_3PO_4$ | 18 | 18—C—6[2] | 75 | 230 | ,, | 1:1 | 3.95 | 1.54 | 5.17 | 1.10 |
| 22 | 3 | PPNI[8] | 3 | TG[3] | 75 | 230 | ,, | 1:1 | 2.33 | .63 | 6.63 | 0.67 |
| 23 | 3 | KI | 3 | $H_2O$ | 75 | 230 | ,, | 1:1 | 4 | .90 | — | 1.32 |
| 24 | 3 | KI | 18 | $H_2O$ | 75 | 230 | ,, | 1:1 | 4 | 1.22 | — | 2.27 |
| 25 | 9 | KI | 30 | THF[4] | 75 | 230 | ,, | 1:1 | 4 | .017 | .215 | — |
| 26 | 3.51 | LiI | 15.9 | BL[5] | 75 | 230 | ,, | 1:1 | 0.9 | .84 | 2.65 | 2.60 |
| 27 | 3 | KI | 18 | 18—C—6[2] | 75 | 230 | ,, | 1:1 | .75 | 1.29 | 6.58 | 0.12 |
| 28 | 9 | KI | 54 | 18—C—6[2] | 75 | 230 | ,, | 1:1 | .22 | 2.07 | 7.88 | 1.22 |
| 29 | 15 | KI | 60 | 18—C—6[2] | 75 | 230 | ,, | 1:1 | .20 | 2.40 | 7.04 | 1.39 |
| 30 | 3 | KI | 18 | NMP[6] | 75 | 230 | ,, | 1:1 | .65 | .20 | 7.15 | 0.15 |
| 31 | 3 | NaI | 18 | Sulf[1] | 75 | 230 | ,, | 1:1 | 1.25 | 1.55 | 4.75 | 0.13 |
| 32 | 9 | NaI | 54 | Sulf[1] | 75 | 200 | ,, | 1:1 | 1.42 | 2.89 | 4.36 | 0.16 |
| 33 | 3 | KOAc[9] | 18 | Sulf[1] | 75 | 230 | ,, | 1:1 | 4 | .40 | 4.30 | — |
| 34 | 9 | KI | 54 | NMP[6] | 75 | 180 | ,, | 1:1 | 1.83 | .41 | 2.68 | — |

TABLE II (Continued)

| Example Nos. | Millimoles of Ruthenium | Lewis base | Millimoles of Lewis base | Solvent | Milliliters of Solvent | Reaction Temperature, °C. | Reaction Pressure, bar | $H_2$/CO Ratio | Reaction Period, hours | Grams of Ethylene Glycol Recovered | Grams of Methanol Recovered | Grams of Ethanol Recovered[11] |
|---|---|---|---|---|---|---|---|---|---|---|---|---|
| 35 | 3 | CsI | 18 | 18–C–6[2] | 75 | 230 | 862 | 1:1 | .70 | 1.15 | 7.83 | 0.88 |
| 36 | 3 | KI | 18 | NMP[6] | 75 | 210 | 1034 | 1:1 | .83 | .27 | 5.69 | – |
| 37 | 3 | KI | 18 | NMP[6] | 75 | 180 | 1379 | 1:1 | 2.17 | .33 | 2.84 | – |
| 38 | 3 | KI | 18 | NMP[6] | 75 | 230 | 862 | 1:1 | .60 | .11 | 7.42 | – |
| 39 | 3 | KI | 18 | 18–C–6[2] | 75 | 230 | ,, | 1:1 | .82 | 1.13 | 9.38 | 1.07 |
| 40 | 3 | PPNI[8] | 18 | NMP[6] | 75 | 230 | ,, | 1:1 | .5 | .23 | 6.91 | .31 |
| 41 | 3 | CsI | 18 | NMP[6] | 75 | 230 | ,, | 1:1 | .63 | .19 | 7.2 | .53 |
| 42 | 3 | NaI | 18 | Sulf[1] | 75 | 230 | ,, | 1:1 | 1.03 | .79 | 3.16 | .17 |
| 43 | 9 | NaI | 54 | Sulf[1] | 75 | 300 | ,, | 1:1 | 1.17 | 2.06 | 3.79 | .16 |
| 44 | 9 | KI | 54 | 18–C–6[2] | 75 | 210 | ,, | 1:1 | .7 | 1.99 | 7.65 | .48 |
| 45 | 15 | KI | 90 | Sulf[1] | 70 | 180 | ,, | 1:1 | 2 | 2.46 | 2.03 | – |
| 46 | 3 | KI | 90 | Sulf[1] | 70 | 180 | ,, | 1:1 | 2 | .31 | .64 | – |
| 47 | 5 | KI | 30 | Sulf[1] | 75 | 180 | ,, | 1:1 | 2 | .66 | .80 | – |
| 48 | 30 | KI | 180 | Sulf[1] | 65 | 180 | ,, | 1:1 | 1.68 | 4.19 | 2.14 | – |
| 49 | 9 | KI | 59 | 18–C–6[2] | 75 | 180 | ,, | 2:1 | 1.95 | 2.41 | 4.91 | – |

TABLE II (Continued)

| Example Nos. | Millimoles of Ruthenium | Lewis base | Millimoles of Lewis base | Solvent | Milliliters of Solvent | Reaction Temperature, °C. | Reaction Pressure, bar | $H_2$/CO Ratio | Reaction Period, hours | Grams of Ethylene Glycol Recovered | Grams of Methanol Recovered | Grams of Ethanol Recovered[11] |
|---|---|---|---|---|---|---|---|---|---|---|---|---|
| 50 | 3 | KI | 18 | Sulf[1] | 75 | 210 | 862 | 2:1 | 2 | 1.34 | 4.10 | — |
| 51 | 3 | KI | 18 | 18—C—6[2] | 75 | 210 | ,, | 2:1 | 1.82 | 1.16 | 8.21 | — |
| 52 | 3 | KI | 18 | Sulf[1] | 75 | 210 | ,, | 1:1 | 2 | 1.39 | 3.40 | — |
| 53 | 3 | KI | 60 | Sulf[1] | 75 | 210 | ,, | 1:1 | 1.25 | 1.36 | 4.32 | — |
| 54 | 9 | KI | 60 | Sulf[1] | 75 | 180 | ,, | 1:1 | 2 | 2.39 | 2.49 | — |
| 55 | 9 | KI | 60 | Sulf[1] | 75 | 180 | ,, | 2:1 | 2 | 2.40 | 3.50 | — |
| 56 | 3 | CsCl | 18 | 18—C—6[2] | 75 | 230 | ,, | 1:1 | 4 | .30 | 5.61 | — |
| 57 | 9 | KI | 54 | 18—C—6[2] | 75 | 200 | ,, | 1:1 | .65 | 1.66 | 6.05 | — |
| 58 | 15 | KI | 60 | 18—C—6[2] | 75 | 230 | ,, | 1:1 | .17 | 1.79 | 6.60 | — |
| 59 | 15 | KI | 60 | 18—C—6[2] | 75 | 260 | ,, | 1:1 | .13 | .65 | 5.37 | 2.90 |
| 60 | 15 | KI | 60 | 18—C—6[2] | 75 | 200 | ,, | 1:1 | .47 | 2.96 | 6.86 | — |
| 61 | 30 | KI | 180 | Sulf[1] | 65 | 230 | ,, | 1:1 | .17 | 2.31 | 5.55 | — |
| 62 | 6 | LiI | 12 | Sulf[1] | 75 | 230 | 552 | 1:1 | 2.03 | 1.11 | 4.55 | 0.74 |
| 63 | 3 | KI | 18 | Sulf[1] | 75 | 230 | 862 | 1:1 | 1.08 | 1.15 | 4.91 | — |
| 64 | 45 | KI | 180 | Sulf[1] | 65 | 230 | ,, | 1:1 | 0.12[7] | 2.44 | 5.2 | .32 |
| 65 | 3 | CsI | 18 | 18—C—6[2] | 75 | 230 | ,, | 1:1 | 0.55 | 0.71 | 5.63 | 0.27 |

TABLE II (Continued)

| Example Nos. | Millimoles of Ruthenium | Lewis base | Millimoles of Lewis base | Solvent | Milliliters of Solvent | Reaction Temperature, °C. | Reaction Pressure, bar | $H_2$/CO Ratio | Reaction Period, hours | Grams of Ethylene Glycol Recovered | Grams of Methanol Recovered | Grams of Ethanol Recovered[11] |
|---|---|---|---|---|---|---|---|---|---|---|---|---|
| 66 | 3 | $BaI_2$ | 18 | 18–C–6[2] | 75 | 230 | 862 | 1:1 | 2.0 | 0.01 | 1.77 | 0.34 |
| 67 | 9 | KI | 54 | 18–C–6[2] | 75 | 260 | 552 | 1:1 | 0.68 | 0.22 | 3.20 | 1.91 |
| 68 | 9 | KI | 54 | 18–C–6[2] | 75 | 280 | ,, | 1:1 | 0.42 | 0.06 | 2.60 | 1.70 |
| 69 | 3 | NaI | 18 | TG[3] | 75 | 280 | ,, | 1:1 | 1.72 | 0.13 | 4.67 | 1.92 |
| 70 | 9 | KOAc[9] | 48 | TG[3] | 75 | 260 | 862 | 1:1 | 2.10 | 0.08 | 7.13 | 1.35 |
| 71 | 3 | CsF | 18 | 18–C–6[2] | 75 | 230 | ,, | 1:1 | 4 | 0.24 | 5.95 | 0.40 |
| 72 | 3 | $K_2CO_3$ | 9 | NMP[6] | 75 | 230 | ,, | 1:1 | 3.45 | 0.35 | 7.24 | 1.45 |
| 73 | 6 | LiI[10] | 12 | Sulf[1] | 75 | 230 | 552 | 1:1 | 3.08 | 0.72 | 2.98 | 0.83 |
| 74 | 30 | KI | 180 | Sulf[1] | 75 | 200 | 414 | 1:1 | 2.0 | 2.41 | 5.5 | 0.1[12] |

[1] Sulfolane.
[2] 18–Crown–6 $[(CH_2CH_2O)_6]$.
[3] Tetraglyme $[CH_3O(CH_2CH_2O)_4CH_3]$.
[4] Tetrahydrofuran.
[5] Gamma-butyrolactone.
[6] N-methylpyrrolidinone.
[7] Rate to ethylene glycol of 5.3 gram moles/liter $hr^{-1}$.
[8] Bis(triphenylphosphine)iminium iodide.
[9] Potassium acetate.
[10] In this example, dicobalt octacarbonyl, $CO_2(CO)_8$ (1 millimole), was added to the reaction mixture as a source of tetracarbonyl cobaltate anion.
[11] A dash mark (—) in this column means that ethanol was not determined quantitatively, although its presence was invariably detected by vapor phase chromatographic analysis.
[12] Approximately.

In some of the examples of Table II, the reaction product was analyzed to determine whether glycerine was present. Each reaction product which was so analyzed was found to contain glycerine. These analyses were made by reacting a sample of the reaction product with bis(trimethylsilyl) trifluoroacetamide. The resulting solution was analyzed by vapor phase chromatography which showed peaks at the correct retention time for the glycerine derivative. The corresponding yields of glycerine were as follows:

| Example No. | Glycerine (grams) |
|---|---|
| 8 | 0.26 |
| 10 | 0.18 |
| 11 | 0.14 |
| 12 | 0.17 |
| 13 | 0.12 |
| 28 | 0.20 |
| 29 | 0.22 |
| 30 | 0.29 |
| 32 | 0.42 |
| 34 | 0.21 |

Example 75

A 500 ml stainless steel bomb reactor containing a removable glass liner was charged with a mixture of 0.50 g. $Ru_3(CO)_{12}$ in 50 mol of glacial acetic acid. Equimolar amounts of carbon monoxide and hydrogen were then added to the reactor to attain a pressure therein of 208 bar at 25°C. The reactor was rocked and the contents heated to 230°C and maintained at this temperature for two hours while rocking the reactor. The reactor was then cooled and vented. The contents of the reactor were removed and analyzed by gas chromatography. This analysis showed that the following products were produced: 3.25 g methyl acetate, 1.71 g ethyl acetate and 0.17 g ethylene glycol diacetate.

Example 76

The procedure of Example 75 was exactly repeated except that the reactor was charged with a mixture of 0.94 g of Ru(acetylacetonate)$_3$ in 50 ml of glacial acetic acid instead of $Ru_3(CO)_{12}$ in 50 ml of glacial acetic acid. Analysis by gas chromatography showed that the following products were produced: 2.77 g methyl acetate and 0.16 g ethylene glycol diacetate.

Example 77

The procedure of Example 75 was exactly repeated except that a mixture of 0.5 g $Ru_3(CO)_{12}$ in 25 ml of glacial acetic acid and 25 ml ethyl acetate was used instead of $Ru_3(CO)_{12}$ in 50 ml of glacial acetic acid. Analysis by gas chromatography showed that the following products were produced: 5.51 g methyl acetate and 0.06 g ethylene glycol diacetate.

Example 78

The procedure of Example 75 was exactly repeated except that a mixture of 0.5 g $Ru_3(CO)_{12}$ in 25 ml of glacial acetic acid and 25 ml of sulfolane was used instead of $Ru_3(CO)_{12}$ in 50 ml of glacial acetic acid. Analysis by gas chromatography showed that the following products were produced: 2.79 g methyl acetate, 0.49 g of ethyl acetate and 0.17 g ethylene glycol diacetate.

Example 79

A reactor, as described in Example 75 was charged with a mixture of 0.50 g $Ru_3(CO)_{12}$ in 50 ml of glacial acetic acid. Equimolar amounts of carbon monoxide and hydrogen were then added to the reactor to attain a pressure therein of 256 bar at 25°C. The reactor was rocked and the contents heated to 230°C and maintained at this temperature for two hours while rocking the reactor. The reactor was cooled and vented. The contents of the reactor were removed and analyzed by gas chromatography. This analysis showed that the following products were produced: 4.22 g methyl acetate and 0.24 g ethylene glycol diacetate.

Example 80

The procedure of Example 75 was exactly repeated except that the reactor was charged with a mixture of 0.50 g $Ru_3(CO)_{12}$, 0.8 ml tributylphosphine and 50 ml glacial acetic acid and the contents were pressurized to 207 bar and heated to 230°C and maintained at 230°C for two hours. Analysis by gas chromatography showed that the following products were produced: 3.25 g methyl acetate, 10.06 g ethyl acetate and 0.04 g ethylene glycol diacetate.

Example 81

The procedure of Example 75 was exactly repeated except that the reactor was charged with a mixture of 0.50 g $Ru_3(CO)_{12}$, 5 ml of $HPF_6.[(C_2H_5)_2O]$ and 50 ml acetic acid and the contents were pressurized to 207 bar and heated to 230°C and maintained at 230°C for two hours. Analysis by gas chromatography showed that the following products were produced: 5.88 g methyl acetate, 8.40 g ethyl acetate and 0.02 g ethylene glycol diacetate.

Example 82

a) In the reactor described above in the examples of Table II (150 ml high pressure autoclave), 100 ml of tetrahydrofuran (THF) was heated at 230°C for four hours under 1550 bar of $H_2/CO$ present in a 1:1 volume ratio. Analysis of the reaction mixture by vapor phase chromatography showed no ethylene glycol and a small amount of methanol.

b) After repeating this procedure, except that 20 mmoles of $Ru_3(CO)_{12}$ was added, analysis of the reaction mixture by vapor-phase chromatography [Tenax®GC (registered trademark of Enka N.V., The Netherlands] column, thermal conductivity detector) showed the major products to be methanol (16.7 area %) and methyl formate (9.7 area %). A peak of 0.56 area % was seen at the correct retention time for ethylene glycol. A sample of this mixture was derivatized by reaction with bis(trimethylsilyl) trifluoroacetamide. Vapor phase chromatography of this mixture showed a peak (0.66 wt. %) at the correct retention time for the ethylene glycol derivative.

c) Experiment b) above was repeated except that a pressure of 1033 bar was used and the reaction was held at 250°C for 13.5 hours. Analysis by vapor-phase chromatography as described in experiment b) showed a peak of 24.5 area % for methanol and a peak of 4.1 area % for methyl formate. A peak of 0.13 area % was observed at the correct retention time for ethylene glycol. After derivatization as in the previous example, a vapor phase chromatographic peak of 0.11 wt. % was observed at the correct retention time for the ethylene glycol derivative.

Example 83

The reactor described above (150 ml stirred high pressure autoclave referred to in the examples of Table II) was charged with 1.4 grams of ruthenium oxide, $RuO_2.xH_2O$ (from Matthey Bishop, Inc., Malvern, Pa., distributed by Alfa Division, Ventron Corp., Andover, Massachusetts) which is characterized as being composed of 53% Ru by weight. Also added were 3.75 g $KHCO_3$, 60 ml of methanol, and 15 ml of $H_2O$. The mixture was heated at 200°C for 4 hours under a pressure of 552 bar of 2/1 volume ratio of $H_2/CO$. No uptake of gas was observed. Analysis of the solution by vapor phase chromatography showed no alcohol products. Particles of Ru metal were observed in the final mixture. An identical experiment was performed using 1.53 g of $Ru_3(CO)_{12}$ instead of $RuO_2.xH_2O$. No gas uptake was observed, and no alcohol products were detected. No ruthenium metal or insoluble particles were observed in the final solution.

Though this example fails to show the production of long chain monohydric alcohols, the absence of those products demonstrates the vagaries of heterogeneous catalysis where the reactions are typically dependent on the source and history of the catalyst employed.

**Claims for the Contracting States: BE FR GB NL DE**

1. The process for selectively making the products methanol, ethylene glycol and ethanol directly from the reaction of hydrogen and carbon monoxide, which comprises:

(a) establishing and maintaining a solvent-containing liquid phase comprising solubilized ruthenium carbonyl complex in which the solvent has a dielectric constant of at least 2, determined at 25°C or at its melting point, whichever is higher;

(b) supplying hydrogen and carbon monoxide in said liquid phase; and

(c) maintaining said liquid phase for a sufficient period of time at a temperature and pressure which causes said hydrogen and carbon monoxide to react to produce such products, said temperature is between 50°C and 400°C, preferably between 100°C and 350°C, and said pressure is between 34.5 bar (500 psia) and 1033 bar (15,000 psia), preferably between 34.5 bar (500 psia) and 862 bar (12,500 psia).

2. The process of claim 1 wherein a promoter of the reaction is provided in the liquid phase.

3. The catalyst composition produced by the process of claim 1 and 2.

4. The process of claim 2 wherein the promoter is a Lewis base containing compound, preferably

an alkali metal halide, especially an alkali metal iodide, e.g. sodium iodide, lithium iodide, potassium iodide, cesium iodide or an alkali metal acetate.

5. The process of claim 2 wherein the solvent acts as a promoter.

6. The process of claim 5 wherein the solvent is a Lewis base containing compound.

7. The process of claim 2 wherein the solvent is polar and is preferably water, a sulfone or a lactam.

8. The process of claim 2 wherein the solvent complexes ions and is preferably an ether, especially a crown ether, an alkyl ether of an alkylene glycol, e.g. a dialkyl ether of a polyalkylene glykol, e.g. tetraglyme, a lactone, e.g. butyrolactone.

9. The process of claim 1 wherein the pressure is the total pressure of hydrogen and carbon monoxide supplied to said process.

10. The process of claim 1 and 2 wherein the carbon monoxide and hydrogen are continuously supplied to the liquid phase and product is removed continuously from said liquid phase in combination with unreacted carbon monoxide and hydrogen.

11. The process of claim 10 wherein unreacted carbon monoxide and hydrogen are recycled to the liquid phase.

12. The process of claim 10 and 11 wherein the amount of promoter provided to the reaction is that amount which achieves a measurable promotional effect.

13. The process of claim 4, 10 and 11 wherein the amount of promoter provided in the liquid phase ranges from 0,1 mole to $10^6$ moles for each gram atom of ruthenium present.

**Claims for the Contracting States: IT SE**

1. The process for selectively making the products methanol, ethylene glycol and ethanol, or carboxylate derivatives thereof, directly from the reaction of hydrogen and carbon monoxide, which comprises:

(a) establishing and maintaining a solvent-containing liquid phase comprising solubilized ruthenium carbonyl complex in which the solvent has a dielectric constant of at least 2, determined at 25°C or at its melting point, whichever is higher;

(b) supplying hydrogen and carbon monoxide in said liquid phase; and

(c) maintaining said liquid phase for a sufficient period of time at a temperature and pressure which causes said hydrogen and carbon monoxide to react to produce such products, said temperature is between 50°C and 400°C, preferably between 100°C and 350°C, and said pressure is between 34,5 bar (500 psia) and 1033 bar (15,000 psia), preferably between 34,5 bar (500 psia) and 862 bar (12,500 psia).

2. The process of claim 1 wherein a promoter of the reaction is provided in the liquid phase.

3. The catalyst composition produced by the process of claim 1 and 2.

4. The process of claim 2 wherein the promoter is a Lewis base containing compound, preferably an alkali metal halide, especially an alkali metal iodide, e.g. sodium iodide, lithium iodide, potassium iodide, cesium iodide or an alkali metal acetate.

5. The process of claim 2 wherein the solvent acts as a promoter.

6. The process of claim 5 wherein the solvent is a Lewis base containing compound.

7. The process of claim 2 wherein the solvent is polar and is preferably water, a sulfone or a lactam.

8. The process of claim 2 wherein the solvent complexes ions and is preferably an ether, especially a crown ether, an alkyl ether of an alkylene glycol, e.g. a dialkyl ether of a polyalkylene glykol, e.g. tetraglyme, a lactone, e.g. butyrolactone.

9. The process of claim 1 wherein the solvent is a carboxylic acid, e.g. acetic acid, and the products formed are corresponding derivative carboxylates.

10. The process of claim 1 wherein the pressure is the total pressure of hydrogen and carbon monoxide supplied to said process.

11. The process of claim 1 and 2 wherein the carbon monoxide and hydrogen are continuously supplied to the liquid phase and product is removed continuously from said liquid phase in combination with unreacted carbon monoxide and hydrogen.

12. The process of claim 11 wherein unreacted carbon monoxide and hydrogen are recycled to the liquid phase.

13. The process of claim 11 and 12 wherein the amount of promoter provided to the reaction is that amount which achieves a measurable promotional effect.

14. The process of claim 4, 11 and 12 wherein the amount of promoter provided in the liquid phase ranges from 0,1 mole to $10^6$ moles for each gram atom of ruthenium present.

**Revendications pour les Etats contractants: BE FR GB NL DE**

1. Procédé pour produire sélectivement du méthanol, de l'éthylène-glycol et de l'éthanol directement par réaction d'hydrogène et d'oxyde de carbone, qui consiste:

(a) à établir et à maintenir une phase liquide contenant un solvant, comprenant un complexe solubilisé de ruthénium-carbonyle, dont le solvant a une constante diélectrique d'au moins 2, déterminée à 25°C ou à son point de fusion, selon la température qui est la plus haute;

(b) à faire arriver de l'hydrogène et de l'oxyde de carbone dans ladite phase liquide; et

(c) à maintenir ladite phase liquide pendant une période suffisante à une température et à une pression qui font réagir l'hydrogène et l'oxyde de carbone pour former lesdits produits, ladite température étant comprise entre 50 et 400°C, de préférence entre 100 et 350°C, et ladite pression absolue étant comprise entre 34,5 bars (500 $lb/in^2$) et 1033 bars (15 000 $lb/in^2$), de préférence entre 34,5 bars (500 $lb/in^2$) et 862 bars (12 500 $lb/in^2$).

2. Procédé suivant la revendication 1, dans lequel un activateur de réaction est prévu dans la phase liquide.

3. La composition de catalyseur produite par le procédé suivant les revendications 1 et 2.

4. Procédé suivant la revendication 2, dans lequel l'activateur est un composé contenant une base de Lewis, de préférence un halogénure de métal alcalin, notamment un iodure de métal alcalin, par exemple l'iodure de sodium, l'iodure de lithium, l'iodure de potassium, l'iodure de césium ou un acétate de métal alcalin.

5. Procédé suivant la revendication 2, dans lequel le solvant agit comme activateur.

6. Procédé suivant la revendication 5, dans lequel le solvant est un composé contenant une base de Lewis.

7. Procédé suivant la revendication 2, dans lequel le solvant est polaire et est de préférence l'eau, une sulfone ou un lactame.

8. Procédé suivant la revendication 2, dans lequel le solvant complexe des ions et est de préférence un éther, notamment un éther en couronne, un éther alkylique d'un alkylène-glycol, par exemple un éther dialkylique d'un polyalkylene-glycol tel que le tétraglyme, une lactone, par exemple la butyrolactone.

9. Procédé suivant la revendication 1, dans lequel la pression est la pression totale d'hydrogène et d'oxyde de carbon fournie audit procédé.

10. Procédé suivant les revendications 1 et 2, dans lequel l'oxyde de carbone et l'hydrogène sont introduits en continu dans la phase liquide et le produit est déchargé en continu de ladite phase liquide conjointement avec l'oxyde de carbone et l'hydrogène n'ayant pas réagi.

11. Procédé suivant la revendication 10, dans lequel l'oxyde de carbone et l'hydrogène n'ayant pas réagi sont recyclés dans la phase liquide.

12. Procédé suivant les revendications 10 et 11, dans lequel la quantité d'activateur introduite dans la réaction est la quantité qui procure un effet activateur mesurable.

13. Procédé suivant les revendications 4, 10 et 11, dans lequel la quantité d'activateur introduite dans la phase liquide va de 0,1 à $10^6$ moles pour chaque atome-gramme de ruthénium présent.

**Revendications pour les Etats contractants: IT SE**

1. Procédé pour produire sélectivement du méthanol, de l'éthylène-glycol et de l'éthanol, ou des carboxylates de ces composés, directement par réaction d'hydrogène et d'oxyde de carbone, qui consiste:

(a) à établir et maintenir une phase liquide contenant un solvant, renfermant un complexe solubilisé de ruthénium-carbonyle, dont le solvant a une constante diélectrique d'au moins 2, déterminée à 25°C ou à son point de fusion selon la température qui est la plus haute;

(b) à faire arriver de l'hydrogène et de l'oxyde de carbone dans ladite phase liquide; et

(c) à maintenir ladite phase liquide pendant une période suffisante à une température et à une pression ayant pour effet de faire réagir l'hydrogène et l'oxyde de carbone pour former lesdits produits, ladite température étant comprise entre 50 et 400°C, de préférence entre 100 et 350°C, et ladite pression absolue étant comprise entre 34,5 bars (500 $lb/in^2$) et 1033 bars (15 000 $lb/in^2$, de préférence entre 34,5 bars (500 $lb/in^2$) et 862 bars (12 500 $lb/in^2$).

2. Procédé suivant la revendication 1, dans lequel l'activateur de la réaction est prévu dans la phase liquide.

3. La composition de catalyseur obtenue par le procédé suivant les revendications 1 et 2.

4. Procédé suivant la revendication 2, dans lequel l'activateur est un composé contenant une base de Lewis, de préférence un halogénure de métal alcalin, notamment un iodure de métal alcalin, par exemple l'iodure de sodium, l'iodure de lithium, l'iodure de potassium, l'iodure de césium ou un acétate de métal alcalin.

5. Procédé suivant la revendication 2, dans lequel le solvant agit comme un activateur.

6. Procédé suivant la revendication 5, dans lequel le solvant est un composé contenant une base de Lewis.

7. Procédé suivant la revendication 2, dans lequel le solvant est polaire et est de préférence l'eau, une sulfone ou un lactame.

8. Procédé suivant la revendication 2, dans lequel le solvant complexe des ions et est de

préférence un éther, notamment un éther en couronne, un éther alkylique d'un alkylène-glycol, par exemple un éther dialkylique d'un polyalkylène-glycol tel que le tétraglyme, une lactone, par exemple la butyrolactone.

9. Procédé suivant la revendication 1, dans lequel le solvant est un acide carboxylique, par exemple l'acide acétique, et les produits formés sont des carboxylates correspondants.

10. Procédé suivant la revendication 1, dans lequel la pression est la pression totale de l'hydrogène et de l'oxyde de carbone introduits dans le procédé.

11. Procédé suivant les revendications 1 et 2, dans lequel l'oxyde de carbone et l'hydrogène sont introduits en continu dans la phase liquide et le produit est déchargé en continu de ladite phase liquide conjointement avec l'oxyde de carbone et l'hydrogène n'ayant pas réagi.

12. Procédé suivant la revendication 11, dans lequel l'oxyde de carbone et l'hydrogène n'ayant pas réagi sont recyclés dans la phase liquide.

13. Procédé suivant les revendications 11 et 12, dans lequel la quantité d'activateur introduite dans la réaction est la quantité qui produite un effet activateur mesurable.

14. Procédé suivant les revendications 4, 11 et 12, dans lequel la quantité d'activateur introduite dans la phase liquide va d'environ 0,1 à $10^6$ moles pour chaque atome-gramme de ruthénium présent.

**Patentansprüche für die Vertraggstaaten: BE FR GB NL DE**

1. Verfahren zur selektiven Herstellung der Produkte Methanol, Ethylenglykol und Ethanol direkt aus der Reaktion von Wasserstoff und Kohlenmonoxid, dadurch gekennzeichnet, daß man

(a) eine lösungsmittelhaltige flüssige Phase, die löslich gemachten Rutheniumcarbonylkomplex enthält, bereitstellt und aufrechterhält, in welcher das Lösungsmittel eine dielektrische Konstante von mindestens 2, bestimmt bei 25°C oder dessen Schmelzpunkt, (wobei der jeweils höhere Wert angewendet wird) hat,

(b) Wasserstoff und Kohlenmonoxid in die flüssige Phase einführt und

(c) die flüssige Phase für eine ausreichende Zeitdauer auf einer Temperatur und einem Druck hält, die eine Reaktion von Wasserstoff und Kohlenmonoxid unter Bildung dieser Produkte bewirken, wobei die Temperatur zwischen 50 und 400°C, vorzugsweise zwischen 100 und 350°C, und der Druck zwischen 34,5 bar (500 psia) und 1033 bar (15 000 psia), vorzugsweise zwischen 34,5 bar (500 psia) und 862 bar (12 500 psia), liegt.

2. Verfahren nach Anspruch 1, dadurch gekennzeichnet, daß in der flüssigen Phase ein Reaktionsbeschleuniger vorgesehen ist.

3. Katalysatorpräparat, hergestellt nach dem Verfahren von Anspruch 1 und 2.

4. Verfahren nach Anspruch 2, dadurch gekennzeichnet, daß der Beschleuniger eine eine Lewis-Base enthaltende Verbindung, vorzugsweise eine Alkalimetallhalogenid, insbesondere ein Alkalimetalljodid, z.B. Natriumjodid, Lithiumjodid, Kaliumjodid, Cäsiumjodid, oder ein Alkalimetallacetat ist.

5. Verfahren nach Anspruch 2, dadurch gekennzeichnet, daß das Lösungsmittel als Beschleuniger wirkt.

6. Verfahren nach Anspruch 5, dadurch gekennzeichnet, daß das Lösungsmittel eine eine Lewis-Base enthaltende Verbindung ist.

7. Verfahren nach Anspruch 2, dadurch gekennzeichnet, daß das Lösungsmittel polar und vorzugsweise Wasser, ein Sulfon oder ein Lactam ist.

8. Verfahren nach Anspruch 2, dadurch gekennzeichnet, daß das Lösungsmittel Ionen komplex bindet und vorzugsweise ein Ether, insbesondere ein Kronenether, ein Alkylether eines Alkylenglykols, z.B. ein Dialkylether eines Polyalkylenglykols, z.B. Tetraglym, ein Lacton, z.B. Butyrolacton, ist.

9. Verfahren nach Anspruch 1, dadurch gekennzeichnet, daß der Druck der auf das Verfahren angelegte Gesamtdruck aus Wasserstoff und Kohlenmonoxid ist.

10. Verfahren nach Anspruch 1 und 2, dadurch gekennzeichnet, daß Kohlenmonoxid und Wasserstoff kontinuierlich in die flüssige Phase eingeführt werden und das Produkt kontinuierlich in Kombination mit nicht umgesetzten Kohlenmonoxid und Wasserstoff aus der flüssigen Phase entfernt wird.

11. Verfahren nach Anspruch 10, dadurch gekennzeichnet, daß nicht umgesetztes Kohlenmonoxid und Wasserstoff zur flüssigen Phase zurückgeführt werden.

12. Verfahren nach Anspruch 10 und 11, dadurch gekennzeichnet, daß die für die Reaktion vorgesehene Beschleunigermenge diejenige ist, die einen meßbaren beschleunigenden Effekt erzielt.

13. Verfahren nach Anspruch 4, 10 und 11, dadurch gekennzeichnet, daß die in der flüssigen Phase vorgesehene Beschleunigermenge zwischen 0,1 Mol bis $10^6$ Mol pro g-Atom des anwesenden Rutheniums beträgt.

**Patentansprüche für die Vertragsstaaten: IT SE**

1. Verfahren zur selektiven Herstellung der Produkte Methanol, Ethylenglykol und Ethanol oder deren Carboxylatderivate direkt aus der Reaktion von Wasserstoff und Kohlenmonoxid, dadurch gekennzeichnet, daß man

(a) eine lösungsmittelhaltige flüssige Phase die löslich gemachten Rutheniumcarbonylkomplex

enthält, bereitstellt und aufrechterhält, in welcher das Lösungsmittel eine dielektrische Konstante von mindestens 2, bestimmt bei 25°C oder dessen Schmelzpunkt, (wobei der jeweils höhere Wert angewendet wird) hat

(b) Wasserstoff und Kohlenmonoxid in die flüssige Phase einführt und

(c) die flüssige Phase für eine ausreichende Zeitdauer auf einer Temperatur und einem Druck hält, die eine Reaktion von Wasserstoff und Kohlenmonoxid unter Bildung dieser Produkte bewirken, wobei die Temperatur zwischen 50 und 400°C, vorzugsweise zwischen 100 und 350°C, und der Druck zwischen 34,5 bar (500 psia) und 1033 bar (15 000 psia), vorzugsweise zwischen 34,5 bar (500 psia) und 862 bar (12 500 psia), liegt.

2. Verfahren nach Anspruch 1, dadurch gekennzeichnet, daß in der flüssigen Phase ein Reaktionsbeschleuniger vorgesehen ist.

3. Katalysatorpräparat, hergestellt nach dem Verfahren von Anspruch 1 und 2.

4. Verfahren nach Anspruch 2, dadurch gekennzeichnet, daß der Beschleuniger eine eine Lewis-Base enthaltende Verbindung, vorzugsweise ein Alkalimetallhalogenid, insbesondere ein Alkalimetalljodid, z.B. Natriumjodid, Lithiumjodid, Kaliumjodid, Cäsiumjodid, oder ein Alkalimetallacetat ist.

5. Verfahren nach Anspruch 2, dadurch gekennzeichnet, daß das Lösungsmittel als Beschleuniger wirkt.

6. Verfahren nach Anspruch 5, dadurch gekennzeichnet, daß das Lösungsmittel eine eine Lewis-Base enthaltende Verbindung ist.

7. Verfahren nach Anspruch 2, dadurch gekennzeichnet, daß das Lösungsmittel polar und vorzugsweise Wasser, ein Sulfon oder ein Lactam ist.

8. Verfahren nach Anspruch 2, dadurch gekennzeichnet, daß das Lösungsmittel Ionen komplex bindet und vorzugsweise ein Ether, insbesondere ein Kronenether, ein Alkylether eines Alkylenglykols, z.B. ein Dialkylether eines Polyalkylenglykols, z.B. Tetraglym, ein Lacton, z.B. Butyrolacton, ist.

9. Verfahren nach Anspruch 1, dadurch gekennzeichnet, daß das Lösungsmittel eine Carbonsäure, z.B. Essigsäure, ist und die gebildeten Produkte die entsprechenden Carboxylatderivate sind.

10. Verfahren nach Anspruch 1, dadurch gekennzeichnet, daß der Druck der auf das Verfahren angelegte Gesamtdruck aus Wasserstoff und Kohlenmonoxid ist.

11. Verfahren nach Anspruch 1 und 2, dadurch gekennzeichnet, daß Kohlenmonoxid und Wasserstoff kontinuierlich in die flüssige Phase eingeführt werden und das Produkt kontinuierlich in Kombination mit nicht umgesetzten Kohlenmonoxid und Wasserstoff aus der flüssigen Phase entfernt wird.

12. Verfahren nach Anspruch 11, dadurch gekennzeichnet, daß nicht umgesetztes Kohlenmonoxid und Wasserstoff zur flüssigen Phase zurückgeführt werden.

13. Verfahren nach Anspruch 11 und 12, dadurch gekennzeichnet, daß die für die Reaktion vorgesehene Beschleunigermenge diejenige ist, die einen meßbaren beschleunigenden Effekt erzielt.

14. Verfahren nach Anspruch 4, 11 und 12, dadurch gekennzeichnet, daß die in der flüssigen Phase vorgesehene Beschleunigermenge zwischen 0,1 Mol bis $10^6$ Mol pro g-Atom des anwesenden Rutheniums beträgt.